# EUROPEAN PATENT APPLICATION

(11) **EP 3 287 028 A1**
(43) Date of publication of application: **28.02.2018**
(21) Application number: 16186010.1
(22) Date of filing: 26.08.2016
(51) Int. Cl.: A41D 13/11, A61K 8/02, A62B 23/02, D06M 11/13, A01N 25/24, A01N 25/34, A01N 33/12, A01N 47/44, A01N 59/16, A61L 2/16, D06M 13/256, D06M 13/352, D06M 16/00

(54) **WASH-DURABLE FACE MASK WITH ANTIMICROBIAL PROPERTIES AND/OR IMPROVED WASHABILITY**

(71) Applicant: Green Impact Holding AG, 6330 Cham/Zug (CH)
(72) Inventor: SWAMY, Sanjeev, 6442 Gersau (CH)
(74) Representative: Karl, Christof

(57) **Abstract**

The present invention is directed to a face mask comprising a multi-layer filter material. One or more antimicrobial and/or hydrophilic and/or stain release agents are adhered to a textile material that forms the outer layers of the multi-layer filter material. The agent(s) is/are adhered to the textile material in such a manner that they are not released from the textile material even if the face mask is washed, so that the face mask as such is re-usable. The particular arrangement of textile layers of the face mask according to the present invention also allows that the face mask can be washed. That is, the textile and the overall structure of the face mask is preserved albeit the physical stress applied during washing. In preferred embodiments, where one or more antimicrobial agents are adhered to the textile material, the face mask can be used to protect the wearer against airborne particulate and microbial pollution, and, in particular, sanitize the inhaled air. Likewise, the environment, such as other persons, are protected from particles and microbes that are exhaled by the wearer of a face mask according to the present invention. Washability and/or usability of the textile material within the multi-layer filter material of the face mask is/are improved when one or more hydrophilic and/or stain release agents are adhered to the textile material, which is particularly advantageous to ensure that the face mask is rendered as clean as possible during wash, i.e. that absorbed or adsorbed stains are released from the textile. The invention further relates to a method of finishing a textile material by applying and binding antimicrobial and/or hydrophilic and/or stain release agents to the textile material so that the agents are essentially irreversibly adhered to the finished textile material.

## Description

### FIELD OF THE INVENTION

The present invention is directed to a face mask comprising a multi-layer filter material. One or more antimicrobial and/or hydrophilic and/or stain release agents are adhered to a textile material that forms the outer layers of the multi-layer filter material. The agent(s) is/are adhered to the textile material in such a manner that they are not released from the textile material even if the face mask is washed, so that the face mask as such is re-usable. The particular arrangement of textile layers of the face mask according to the present invention also allows that the face mask can be washed. That is, the textile and the overall structure of the face mask is preserved albeit the physical stress applied during washing. In preferred embodiments, where one or more antimicrobial agents are adhered to the textile material, the face mask can be used to protect the wearer against airborne particulate and microbial pollution, and, in particular, sanitize the inhaled air. Likewise, the environment, such as other persons, are protected from particles and microbes that are exhaled by the wearer of a face mask according to the present invention. Washability and/or usability of the textile material within the multi-layer filter material of the face mask is/are improved when one or more hydrophilic and/or stain release agents are adhered to the textile material, which is particularly advantageous to ensure that the face mask is rendered as clean as possible during wash, i.e. that absorbed or adsorbed stains are released from the textile. The invention further relates to a method of finishing a textile material by applying and binding antimicrobial and/or hydrophilic and/or stain release agents to the textile material so that the agents are essentially irreversibly adhered to the finished textile material.

### BACKGROUND OF THE INVENTION

Particulate filtering face masks are used in various circumstances to protect against a variety of airborne particles and contaminations, such as microbes that are transferred via droplets, fine particles and dust. Face masks are regularly used in hospitals and industry environments as well as in other polluted environments, such as in larger cities with high air pollution. Here, the danger of infections and threats to the respiratory system are high due to microbial contamination and/or fine particle pollution. Workers in the health care system as well as treated patients are particularly exposed to infectious contaminations and therefore require high standards of disinfection routines to prevent spread of pathogens within the health care system as well as population-wide crisis situations, such as the recent H1N1 pandemic.

Most commonly used face masks comprise particulate filtering materials that trap particles, such as microorganisms, out of the breathing air.

Respiratory face masks produced from particulate filtering materials reduce the risk of infections for the face mask's wearer, however they are typically disposable, i.e. non-washable and not re-usable. Other face masks are washable and re-usable, however, they are not antimicrobial, i.e. they - at best - filter microbes from air passing through the mask, but they do not kill the filtered microbes. Therefore, such filtered microbes stay infectious within and on such known re-usable face masks.

US patent application US 11/757336 tries to overcome the problems of the prior art face masks by providing a respiratory face mask which is self-sanitizing. The mask comprises a particulate filter layer to trap particles and at least one porous, continuously sanitizing layer located adjacent to or sandwiching the filter layer. The sanitizing effect is achieved by releasing free silver and/or copper ions from the porous layer into the filter layer. The metal ions may be attached to the porous layer by use of zeolite carriers. The face mask as such is claimed to be washable and re-usable while retaining its antimicrobial activity and/or filtration capability. With reference to three different US patent documents, the lowest amount of attached silver and/or copper ions or the zeolite of silver is 0.1% to 20% by weight of fiber of the sanitizing layer (reference to US 09/565138). Optionally, the antimicrobial compounds may be produced, coated, or impregnated with the sanitizing layer. The wash-durability is not demonstrated in said US patent application. Also, the face mask described in the US patent application is expensive in production, in particular due to the very high amount of silver ions employed to the fibers of the mask.

US patent application US 11/302644 provides a protective, antimicrobial article comprising a nonwoven fiber web that is at least partially coated with a stable, non-leaching antimicrobial or antiviral composition. Such composition may include polyhexamethylene biguanide, chitosan and a combination of a chaotropic surfactant, polyols, copper oxide, and an organic acid. Due to the chemical and mechanical stability of the antimicrobial coating, the inventors claim that the antimicrobial coating would not leach from the substrate surface upon which it is applied. The antimicrobial coating is, however, not wash-durable.

US patent application US 12/675336 describes an antimicrobial fiber comprising an antimicrobial composition of at least two antimicrobial agents, which are of organic origin, such as Triclosan™, and inorganic origin, such as silver-zinc-glass, and a hydrophilic modifier, such as Irgasurf™ HL560. Due to dispersion of the antimicrobial agents and the hydrophilic modifier within the fiber, they do not leach off during the typical and reasonable conditions of use. However, the described antimicrobial fiber is not wash-durable, and a face mask from such fiber not be re-usable.

### SUMMARY OF THE INVENTION

It is an object of the present invention to provide a textile material that overcomes some or all problems of the above-mentioned prior art documents. In particular, one object of the invention is to provide textile materials that can be used for sanitizing air passing through the textile material. Said antimicrobial or sanitizing effect of the textile material shall be preserved even after several washes of the textile material. More generally is it an object of the invention that the textile materials can kill microbes or inhibit their growth as completely as possible. Furthermore, it is an object of the invention that the textile materials are used in a multi-layer filter material, wherein layers of the textile materials sandwich particulate filter material layers. It is a further object of the invention that the multi-layer filter material is used in a face mask, which is easily washable and/or re-usable.

Some or all of these objects are solved by the subject matter of the independent claims. Preferred embodiments are subject of the dependent claims.

In accordance with the present invention, a textile material is provided to which one or more chemical agents are adhered, which chemical agents convey antimicrobial and/or hydrophilic and/or stain release properties to the textile material, in a wash-durable manner. The textile material can be used in a multi-layer filter material which in turn can be used in a face mask. The invention further provides finishing methods for manufacturing the textile materials, by applying liquor application processes such as padding and exhaust processes, followed by heat treatment.

Unless otherwise stated, all percentages hereinafter refer to the ratio of the uptaken weight of antimicrobial agent on a textile and the weight of that fabric without the uptaken antimicrobial agent. The term "on weight fabric" refers to this ratio. Abbreviations are "owf"' or "o.w.f". The term "gpl" means "grams per liter" and is typically used to define the concentration of a substance in a liquor.

In the context of the present invention the terms "textile" and "textile material" relate to a flexible material consisting of fibres, or a network of natural and/or artificial fibres, such as a yarn or a fabric. The material may be in its natural or processed or even finished form. The term "starting textile material" refers to a textile material which has not yet been treated by the finishing processes described in the present disclosure.

Particulate filtering materials are tested and classified according to their filter efficiency towards the smallest-sized particle. Ninety-five percent ("95-rated") is the minimal level of filtration that is approved by the National Institute for Occupational Safety and Health (NIOSH) in the United States. The particulate filtering materials are further classified according to their oil resistance, with "N" indicating no oil resistance, "R" slight oil resistance, and "P" strong oil resistance (for example: a N95-rated, R95-rated or P95-rated particulate filtering material).

The term "antimicrobial" as used in the context of the present invention relates to the ability to kill at least some types of microorganisms, or to inhibit the growth or reproduction of at least some types of microorganisms. Said term relates to any compound, agent, product or process that is harmful to one or more "microorganism" as used in the context of the present invention. Preferably, the one or more "microorganisms" get killed by the "antimicrobial" product or process. By "antimicrobial agent" is meant any substance or combination of substances that kills or prevents the growth of a microorganism. The terms "microorganism" and "microbe", which are used interchangeably in the context of the present invention, are defined to comprise any organism too small to be seen by the unaided eye, such as, especially, single-celled organisms. In particular, the terms "microorganism" and "microbe" cover prokaryotes including bacteria and archaea, eukaryotes including protists, animals like dust mites or spider mites, fungi, and plants like green algae, as well as viruses.

The term "hydrophilic" essentially relates to the attraction of water. In the context of the present invention, the term "hydrophilic" commonly implies attachment or adhering of aqueous liquids at the surface and in the interior of a textile material. Thus, the term "hydrophilic" or "hydrophilic properties" as used herein includes the attachment or adhering of aqueous liquids at or near the surface of a textile material and/or in the interior of a textile material. When the term "hydrophilic" or "hydrophilic properties" is used herein, it means the attachment or adhering of liquid at or near the surface of a textile material. A hydrophilic textile is typically not water-repellent and to the contrary absorbs water quickly. It typically also exhibits good wicking properties. A "hydrophilic agent" is an agent which when applied to a substrate like a textile material, noticeably improves the hydrophilic properties of the substrate.

The term "stain release" relates to easy removal of stains. In the context of the present invention, the term "stain release properties" of a textile commonly refers to its ability of both adsorbing in particular oily stains on the outside surface or on internal surfaces within a textile material and easily removing stains from these surfaces, for instance during laundry. The term "stain release properties" is not to be confused with the term "stain repellent properties" of a textile. Whereas textiles with good stain repellent properties do not adsorb or absorb oily stains in the first place, textiles with good stain release properties do adsorb them but subsequently release them easily. A "stain release agent" is an agent which, when applied to a substrate like a textile material, noticeably improves the stain release properties of that substrate.

Whenever a temperature is mentioned in the present specification, the temperature refers to a temperature applied at normal pressure (101.325 Pa). If in an implementation of the invention higher or lower pressure is applied, the temperatures are understood to be adapted accordingly.

A 1^{st} embodiment of the present invention is a textile material to which one or more stain release agents and/or one or more hydrophilic agents and/or one or more antimicrobial agents are adhered.

A 2^{nd} embodiment of the present invention is a textile material to which one or more stain release agents and one or more hydrophilic agents and preferably one or more antimicrobial agents are adhered.

A 3^{rd} embodiment of the present invention is a textile material to which one or more stain release agents and one or more antimicrobial agents and preferably one or more hydrophilic agents are adhered.

A 4^{th} embodiment of the present invention is a textile material to which one or more antimicrobial agents and one or more hydrophilic agents and preferably one or more stain release agents are adhered.

A 5^{th} embodiment of the present invention is the textile material of any one of the 1^{st} to 4^{th} embodiments, wherein the one or more antimicrobial agents comprise
- at least one, preferably at least two, more preferably at least three, even more preferably at least four, or all five selected from the group consisting of a quaternary ammonium organosilane compound, metal, an azole-based compound, polyglucosamine, and polyhexamethylene biguanide; or
- at least one, preferably at least two, more preferably at least three, and most preferably all four selected from the group consisting of metal, an azole-based compound, polyglucosamine, and polyhexamethylene biguanide; or
- at least one, preferably at least two, more preferably all three selected from the group consisting of metal, an azole-based compound, and a quaternary ammonium organosilane compound; or
- at least metal and at least one selected from the group consisting of an azole-based compound, a quaternary ammonium organosilane compound, polyglucosamine, and polyhexamethylene biguanide; or at least an azole-based compound and at least one selected from the group consisting of a quaternary ammonium organosilane compound, polyglucosamine, and polyhexamethylene biguanide; or at least a quaternary ammonium organosilane compound and at least one selected from the group consisting of polyglucosamine and polyhexamethylene biguanide; or at least polyglucosamine and polyhexamethylene biguanide.

A 6^{th} embodiment of the present invention is the textile material of any one of the 1^{st} to 4^{th} embodiments, wherein the one or more antimicrobial agents comprise
- metal and at least one, or at least two, or all three selected from the group consisting of polyhexamethylene biguanide, polyglucosamine, and an azole-based compound; or
- an azole-based compound and at least one, or at least two, or all three selected from the group consisting of polyhexamethylene biguanide, polyglucosamine, and metal; or
- polyhexamethylene biguanide and at least one, or at least two, or all three selected from the group consisting of metal, polyglucosamine, and an azole-based compound; or
- polyglucosamine and at least one, or at least two, or all three selected from the group consisting of silver cations, polyhexamethylene biguanide, and an azole-based compound; or
- quaternary ammonium organosilane and at least one, preferably both selected from the group consisting of metal and an azole-based compound; or
- metal and at least one, preferably both selected from the group consisting of a quaternary ammonium organosilane compound and an azole-based compound; or
- an azole-based compound and at least one, preferably both selected from the group consisting of quaternary ammonium organosilane and metal.

A 7^{th} embodiment of the present invention is the textile material of any one of the 5^{st} or 6^{th} embodiment, wherein the metal is copper, zinc, or preferably silver, more preferably silver cations.

An 8^{th} embodiment of the present invention is the textile material of any one of the 5^{st} or 6^{th} embodiment, wherein the azole-based compound is thiabendazole or a triazole-based compound.

A 9^{th} embodiment of the present invention is the textile material of the 8^{th} embodiment, wherein the triazole-based compound is propiconazole.

A 10^{th} embodiment of the present invention is the textile material of any one of the 1^{st} to 9^{th} embodiments, wherein the one or more antimicrobial agents are not in the form of nanoparticles.

An 11^{th} embodiment of the present invention is the textile material of any one of the 1^{st} to 10^{th} embodiments, wherein the one or more antimicrobial agents have a particle size, in all dimensions (length, width, height), of at least 250 nanometers, preferably at least 500 nanometers, more preferably at least 750 nanometers, and most preferably at least 1,000 nanometers.

A 12^{th} embodiment of the present invention is the textile material of any one of the 1^{st} to 11^{th} embodiments, wherein the one or more antimicrobial agents are nonionic or cationic.

A 13^{th} embodiment of the present invention is the textile material of any one of the 1^{st} to 12^{th} embodiments, wherein the one or more antimicrobial agents are bound to the textile material either directly, in particular if the agent is a quaternary ammonium organosilane compound, polyglucosamine, or polyhexamethylene biguanide, or by means of an inorganic or organic matrix directly bound to the textile material, in particular if the agent is metal, or via cross linking, in particular if the agent is an azole-based compound.

A 14^{th} embodiment of the present invention is the textile material of any one of the 1^{st} to 13^{th} embodiments, wherein one or more of the one or more antimicrobial agents are bound to the textile material without the cyclodextrin and/or an inclusion complex, in particular without an inclusion complex of fiber-reactive cyclodextrin derivatives and antimicrobial agents.

A 15^{th} embodiment of the present invention is the textile material of any one of the preceding embodiments, wherein the total amount of the one or more antimicrobial agents adhered to the textile material is at most 4.0%, preferably at most 3.0%, more preferably at most 2.5%, and most preferably at most about 2.0% on weight fabric of the textile material.

A 16^{th} embodiment of the present invention is the textile material of any one of the preceding embodiments, wherein the total amount of the one or more antimicrobial agents adhered to the textile material is at least 0.1%, preferably at least 0.2%, more preferably at least 0.3%, and most preferably at least 0.4% on weight fabric of the textile material.

A 17^{th} embodiment of the present invention is the textile material of any one of the 5^{th} to 16^{th} embodiments, wherein the polyhexamethylene biguanide is adhered to the textile material in an amount of at most 1.0%, preferably at most 0.8%, more preferably at most 0.6%, particularly at most 0.5%, and most preferably at most about 0.4% on weight fabric of the textile material.

A 18^{th} embodiment of the present invention is the textile material of any one of the 5^{th} to 17^{th} embodiments, wherein the polyhexamethylene biguanide is adhered to the textile material in an amount of at least 0.1%, preferably at least 0.2%, more preferably at least 0.3%, and most preferably at least about 0.4% on weight fabric of the textile material.

A 19^{th} embodiment of the present invention is the textile material of any one of the 5^{th} to 18^{th} embodiments, wherein the metal is adhered to the textile material in an amount of at most 0.14%, preferably at most 0.12%, more preferably at most 0.10%, particularly at most 0.08% and most preferably at most 0.06% on weight fabric of the textile material.

A 20^{th} embodiment of the present invention is the textile material of any one of the 5^{th} to 19^{th} embodiments, wherein the metal is adhered to the textile material in an amount of at least 0.0005%, preferably at least 0.001%, more preferably at least 0.002%, and most preferably at least about 0.005% on weight fabric of the textile material.

A 21^{st} embodiment of the present invention is the textile material of any one of the 5^{th} to 20^{th} embodiments, wherein the azole-based compound is adhered to the textile material in an amount of at most 1%, preferably at most 0.8%, more preferably at most 0.5%, and most preferably at most about 0.3% on weight fabric of the textile material.

A 22^{nd} embodiment of the present invention is the textile material of any one of the 5^{th} to 21^{st} embodiments, wherein the azole-based compound is adhered to the textile material in an amount of at least 0.005%, preferably at least 0.01%, more preferably at least 0.02%, particularly at least 0.05%, and most preferably at least 0.1% on weight fabric of the textile material.

A 23^{rd} embodiment of the present invention is the textile material of any one of the 5^{th} to 22^{nd} embodiments, wherein the quaternary ammonium organosilane compound is adhered to the textile material in an amount of at most 1.5%, preferably at most 1.25%, more preferably at most 1%, in particular at most 0.8 %, and most preferably at most about 0.6% on weight fabric of the textile material.

A 24^{th} embodiment of the present invention is the textile material of any one of the 5^{th} to 23^{rd} embodiments, wherein the quaternary ammonium organosilane compound is adhered to the textile material in an amount of at least 0.01%, preferably at least 0.025%, more preferably at least 0.05%, and most preferably at least about 0.1% on weight fabric of the textile material.

A 25^{th} embodiment of the present invention is the textile material of any one of the 5^{th} to 24^{th} embodiments, wherein the polyglucosamine is adhered to the textile material in an amount of at most 1.5%, more preferably at most 1.0%, particularly at most 0.6%, most preferably at most about 0.3% on weight fabric of the textile material.

A 26^{th} embodiment of the present invention is the textile material of any one of the 5^{th} to 25^{th} embodiments, wherein the polyglucosamine is adhered to the textile material in an amount of at least 0.01%, preferably at least 0.025%, more preferably at least 0.05%, particularly at least 0.075%, and most preferably at least about 0.1% on weight fabric of the textile material.

A 27^{th} embodiment of the present invention is the textile material of any one of the preceding embodiments, wherein the one or more antimicrobial agents adhered to the textile material increase the reduction value and/or the textile material exhibits a reduction value of *Escherichia coli* ATCC 25922 and/or *Staphylococcus aureus* ATCC 6538 and/or *Pseudomonas aeroginosa* ATCC 15442 and/or *Salmonella enterica* ATCC 10708 and/or *Candida albicans* ATCC 10231 and/or *Aspergillus niger* 16404 measured in accordance with AATCC test method 100-2012 and/or ASTM E2149-10 by/of at least 90% (1 log), preferably at least 99% (2 log), more preferably at least 99.9% (3 log), and most preferably at least 99.99% (4 log), within 1 hour of contact time, preferably within 15 minutes of contact time and/or of at least 99.% (2 log), preferably at least 99.9% (3 log), more preferably at least 99.99% (4 log), most preferably at least 99.999% (5 log) within 24 hours of contact time.

A 28^{th} embodiment of the present invention is the textile material of any one of the preceding embodiments, wherein the one or more hydrophilic agents adhered to the textile material comprise at least one selected from the group consisting of carboxylic acid esters, polyester ether copolymers, starch based emulsions, preferably fatty alcohol ethoxylates, and organosilane terpolymers, preferably fatty alcohol ethoxylates or organosilane terpolymers, most preferably organosilane terpolymers.

A 29^{th} embodiment of the present invention is the textile material of any one of the preceding embodiments, wherein the one or more stain release agents adhered to the textile material comprise at least one selected from the group consisting of fatty alcohol ethoxylates, and organosilane terpolymers, preferably organosilane terpolymers.

A 30^{th} embodiment of the present invention is the textile material of any one of the preceding embodiments, wherein one or more or all of the hydrophilic agents is/are (a) stain release agent(s).

A 31^{st} embodiment of the present invention is the textile material of any one of the preceding embodiments, wherein one or more or all of the stain release agents is/are (a) hydrophilic agent(s).

A ₃₂^{nd} embodiment of the present invention is the textile material of any one of the preceding embodiments, wherein the hydrophilic and/or stain release agents are adhered to the textile material in an amount of together at most 5%, preferably at most 4%, more preferably at most 4%, even more preferably at most 3%, in particular at most 2%, and most preferably at most about 1.5% on weight fabric of the textile material.

A 33^{rd} embodiment of the present invention is the textile material of any one of the preceding embodiments, wherein the hydrophilic and/or stain release agents are adhered to the textile material in an amount of together at least 0.1%, preferably at least 0.25%, more preferably at least 0.5%, particularly at least 0.75%, and most preferably at least about 0.9% on weight fabric of the textile material.

A 34^{th} embodiment of the present invention is the textile material of any one of the 28^{th} or 29^{th} embodiments, or any one of the 30^{th} to 33^{rd} embodiments when dependent from any one of the 28^{th} or 29^{th} embodiments, wherein the organosilane terpolymers are adhered to the textile material in an amount of at least 0.1%, preferably at least 0.2%, more preferably at least 0.5%, particularly at least 0.75%, and most preferably at least about 1% on weight fabric of the textile material.

A 35^{th} embodiment of the present invention is the textile material of any one of the 28^{th} or 29^{th} embodiments, or any one of the 30^{th} to 34^{th} embodiments when dependent from any one of the 28^{th} or 29^{tn} embodiments, wherein the fatty alcohol ethoxylates are adhered to the textile material in an amount of at most 4%, preferably at most 3%, more preferably at most 2%, even more preferably at most 1.5%, and most preferably at most about 1.0% on weight fabric of the textile material.

A 36^{th} embodiment of the present invention is the textile material of any one of the preceding embodiments, wherein the one or more hydrophilic agents adhered to the textile material reduce the water absorbency time by at least 20%, preferably at least 40%, more preferably at least 60%, and most preferably at least 80%, preferably when measured in accordance with AATCC test method 79-2014 (Option A).

A 37^{th} embodiment of the present invention is the textile material of any one of the preceding embodiments, exhibiting a water absorbency time of at most 3 seconds, preferably at most 2 seconds, more preferably at most 1 second, and most preferably at most 0.5 seconds, preferably when measured in accordance with AATCC test method 79-2014 (Option A).

A 38^{th} embodiment of the present invention is the textile material of any one of the preceding embodiments, wherein the one or more hydrophilic agents adhered to the textile material do not increase the water repellency of the textile material, more preferably decrease the water repellency, and most preferably decrease the water repellency by at least one rating, measured in accordance with AATCC test method 22-2014.

A 39^{th} embodiment of the present invention is the textile material of any one of the preceding embodiments, exhibiting a water repellency rating of at most 50, preferably o, measured in accordance with AATCC test method 22-2014.

A 40^{th} embodiment of the present invention is the textile material of any one of the preceding embodiments, wherein the one or more hydrophilic agents adhered to the textile material increase the vertical wicking characteristics by at least a 25%, preferably at least 50%, and most preferably at least 100%, when tested according to AATCC test method 197-2013.

A 41^{st} embodiment of the present invention is the textile material of any one of the preceding embodiments, exhibiting a vertical wicking characteristics of at least 0.1 mm/sec, preferably at least 0.2 mm/sec, more preferably at least 0.3 mm/sec, even more preferably at least 0.4 mm/sec, particularly at least 0.5 mm/sec, and most preferably at least 0.6 mm/sec, when tested according to AATCC test method 197-2013.

A 42^{nd} embodiment of the present invention is the textile material of any one of the preceding embodiments, wherein the one or more hydrophilic agents adhered to the textile material increase the horizontal wicking characteristics by at least 50%, preferably 100%, more preferably 300% and most preferably 500% when tested according to AATCC test method 198-2013.

A 43^{rd} embodiment of the present invention is the textile material of any one of the preceding embodiments, exhibiting a horizontal wicking characteristics of at least 20 mm²/sec, preferably at least 40 mm²/sec, more preferably at least 60 mm²/sec, and most preferably at least 80 mm²/sec, when tested according to AATCC test method 197-2013.

A 44^{th} embodiment of the present invention is the textile material of any one of the preceding embodiments, wherein the one or more stain release agents adhered to the textile material increase the stain release capability of the textile material by at least one rating, more preferably by at least two ratings, particularly at least three ratings, and most preferably at least 4 ratings, when tested according to AATCC test method 130-2010.

A 45^{th} embodiment of the present invention is the textile material of any one of the preceding embodiments, having a stain release capability, measured in accordance with AATCC test method 130-2010, of at least rating 3, preferably at least rating 4, and most preferably rating 5.

A 46^{th} embodiment of the present invention is the textile material of any one of the preceding embodiments, wherein the one or more stain release agents adhered to the textile material increases the stain/oil repellency of the textile material, measured in accordance with AATCC test method 118-2013, by at most one rating, preferably does not increase the stain/oil repellency, more preferably decreases the stain/oil repellency, and most preferably decreases the stain/oil repellency by at least one rating.

A 47^{th} embodiment of the present invention is the textile material of any one of the preceding embodiments, wherein the textile material exhibits a stain/oil repellency, measured in accordance with AATCC test method 118-2013, of at most rating 1, preferably rating o.

A 48^{th} embodiment of the present invention is the textile material of any one of the 1^{st} to 42^{nd} embodiments, wherein the claimed properties of the textile material, such as stain release capability, antimicrobial efficiency, and/or hydrophilicity are achieved even after at least 25 laundry washes in a laundry washing machine at 85±15 °C for 10-15 minutes, preferably using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

A 49^{th} embodiment of the present invention is the textile material of any one of the 1^{st} to 42^{nd} embodiments, wherein the one or more stain release agents and/or the one or more hydrophilic agents and/or the one or more antimicrobial agents are adhered to the textile material in a non-leaching manner.

A 50^{th} embodiment of the present invention is the textile material of any one of the 1^{st} to 42^{nd} embodiments, wherein the starting textile material comprises hydroxyl, peptide and/or carbonyl groups, in particular hydroxyl and/or peptide.

A 51^{st} embodiment of the present invention is the textile material of any one of the preceding embodiments, wherein the starting textile material is a cellulosic textile material, a synthetic textile material, or a blend of cellulosic textile material and synthetic textile material.

A 52^{nd} embodiment of the present invention is the textile material of the 51^{st} embodiment, wherein the cellulosic and/or synthetic textile materials comprise functional groups having the ability to bond one or more antimicrobial agents to the textile material.

A 53^{rd} embodiment of the present invention is the textile material of any one of the 51^{st} or 52^{nd} embodiments, wherein the cellulosic textile material comprises cotton, viscose, rayon, linen, hemp, ramie, jute, and combinations (blends) thereof, preferably viscose.

A 54^{th} embodiment of the present invention is the textile material of any one of the 51^{st} to 53^{rd} embodiments, wherein the synthetic textile material comprises one or more selected from the group consisting of polyester, polyamide (nylon), acrylic polyester, spandex (elastane, Lycra), aramids, modal, sulfar, polylactide (PLA), lyocell, polybutyl tetrachloride (PBT), and combinations (blends) thereof, preferably polyester or polyamide (nylon), most preferably polyester.

A 55^{th} embodiment of the present invention is the textile material of any one of the 51^{st} to 54^{th} embodiments, wherein the blend of cellulosic textile material and synthetic textile material comprises between 20% and 80% of cellulosic textile material, preferably between 30% and 75%, more preferably between 50% and 70%, and most preferably about 65%.

A 56^{th} embodiment of the present invention is the textile material of any one of the 51^{st} to 55^{th} embodiments, wherein the blend of cellulosic textile material and synthetic textile material comprises between 20 and 80% of synthetic textile material, preferably between 25 and 70%, more preferably between 30 and 50%, and most preferably about 35%.

A 57^{th} embodiment of the present invention is the textile material of any one of the 51^{st} to 56^{th} embodiments, wherein the textile material is a fiber, a yarn, or a fabric, preferably a yarn or a fabric, more preferably a fabric.

A 58^{th} embodiment of the present invention is the textile material of the 57^{th} embodiment, wherein the fabric is selected from the group consisting of woven, knitted, warp-knitted, crocheted, and non-woven fabrics, preferably from a woven fabric.

A 59^{th} embodiment of the present invention is the textile material of any one of the preceding embodiments, having a water holding capacity of at least 5 times its weight, preferably at least 7 times its weight, more preferably at least 8 times its weight, in particular at least 10 times its weight, most preferably at least 12 times its weight, preferably when tested according to ASTM D7367-14.

A 60^{th} embodiment of the present invention is a process for finishing a textile material comprising the steps of:
- treating the textile material using an antimicrobial liquor application process like an exhaustion or padding process, wherein the liquor comprises one or more antimicrobial agents;
- preferably drying the textile material and treating the textile material using a hydrophilic/stain release liquor application process like an exhaustion or preferably a padding process, wherein the liquor comprises one or more hydrophilic agents and/or stain release agents; and
- subjecting the treated textile material to a heat treatment.

A 61^{st} embodiment of the present invention is a process for finishing a textile material comprising the steps of:
- preferably treating the textile material using an antimicrobial liquor application process like an exhaustion or padding process, wherein the liquor comprises one or more antimicrobial agents and drying the textile material;
- treating the textile material using a hydrophilic/stain release liquor application process like an exhaustion or preferably a padding process, wherein the liquor comprises one or more hydrophilic agents and/or stain release agents; and
- subjecting the treated textile material to a heat treatment.

A 62^{nd} embodiment of the present invention is the process of the 60^{th} embodiment, wherein the liquor of the antimicrobial and/or hydrophilic/stain release liquor application process has a pH-value of at most 6.9, preferably at most 6.5, more preferably at most 6.3, in particular at most 6.0, and most preferably at most about 5.5, and a pH-value of at least 3.0, preferably at least 3.5, more preferably at least 4.0, even more preferably at least 4.5, in particular at least 5.0, and most preferably at least about 5.5.

A 63^{rd} embodiment of the present invention is the process of any one of the 60^{th} or 62^{nd} embodiments, wherein the amount of stain release and/or hydrophilic agents in the liquor of the antimicrobial liquor application process is together at most 10 gpl, preferably at most 5 gpl, more preferably at most 2 gpl, even more preferably at most 1 gpl, and most preferably o.

A 64^{th} embodiment of the present invention is the process of any one of the 60^{th} or 63^{rd} embodiments, wherein in the antimicrobial and/or the hydrophilic/stain release liquor application process, the agents and preferably all other components in the liquor are dissolved in the liquor, in particular are not dispersed in the liquor, and/or the liquor is substantially free of solids.

A 65^{th} embodiment of the present invention is the process of any one of the 1^{st} to 64^{th} embodiments, wherein the liquor of the antimicrobial and/or hydrophilic/stain release liquor application process contains a solvent, preferably water, and the agents and the solvent form a homogenous mixture, and in particular do not form a slurry or dispersion.

A 66^{th} embodiment of the present invention is the process of any one of the 60^{th} to 65^{th} embodiments, wherein the value of dynamic viscosity of the liquor of the antimicrobial and/or hydrophilic/stain release liquor application process at 20°C and/or 80 °C, in centipoise (cP), is at most 20% higher than the dynamic viscosity of water at 20°C and/or 80 °C, respectively, preferably at most 10%, more preferably at most 5%, particularly at most 2%, and most preferably at most about 0%.

A 67^{th} embodiment of the present invention is the process of any one of the 60^{th} to 66^{th} embodiments, wherein the amount of latex in the liquor of the antimicrobial and/or the hydrophilic/stain release liquor application process is at most 10 gpl, preferably at most 5 gpl, more preferably at most 2 gpl, even more preferably at most 1 gpl, and most preferably o.

A 68^{th} embodiment of the present invention is the process of any one of the 60^{th} to 66^{th} embodiments, wherein the amount of cyclodextrin and/or inclusion complexes, in particular inclusion complexes of fiber-reactive cyclodextrin derivatives and antimicrobial agents in the liquor of the antimicrobial and/or the hydrophilic/stain release liquor application process is at most 10 gpl, preferably at most 5 gpl, more preferably at most 2 gpl, even more preferably at most 1 gpl, and most preferably o.

A 69^{th} embodiment of the present invention is the process of any one of the 60^{th} to 68^{th} embodiments, wherein the amount of dye in the liquor of the antimicrobial and/or main process cycle is at most 10 gpl, preferably at most 5 gpl, more preferably at most 2 gpl, even more preferably at most gpl, and most preferably o.

A 70^{th} embodiment of the present invention is the process of any one of the 60^{th} to 69^{th} embodiments, wherein the drying before the hydrophilic/stain release liquor application process is conducted at least partially at an ambient temperature of at least 70 °C., preferably at least 100 °C, more preferably at least 110°C, most preferably at least about 120°C.

A 71^{st} embodiment of the present invention is the process of any one of the 60^{th} to 70^{th} embodiments, wherein in the drying before the hydrophilic/stain release liquor application process is conducted at an ambient temperature of at most 160 °C,, preferably of at most 140°C, more preferably of at most 130 °C, and most preferably of at most about 120 °C.

A 72^{nd} embodiment of the present invention is the process of any one of the 60^{th} to 71^{st} embodiments, wherein the heat treatment comprises drying conducted at least partially at an ambient temperature of at least 60 °C, in particular at least 100 °C, preferably at least 110 °C, more preferably at least 120 °C.

A 73^{rd} embodiment of the present invention is the process of any one of the 60^{th} to 72^{nd} embodiments, wherein the heat treatment comprises curing of the textile material, wherein the curing is conducted at least partially at a curing ambient temperature of at least 140°C, preferably at least 160°C, more preferably at least 170°C, particularly at least 175 °C, and most preferably at least about 180 °C.

A 74^{th} embodiment of the present invention is the process of any one of the 60^{th} to 73^{rd} embodiments, wherein the heat treatment is conducted at an ambient temperature of at most 200 °C, preferably at most 190 °C, more preferably at most 185 °C, and most preferably at most about 180 °C.

A 75^{th} embodiment of the present invention is the process of any one of the 73^{rd} or 74^{th} embodiments, wherein curing takes place at the curing temperature as defined in the 73^{rd} embodiment over a period of at least 30 seconds, preferably at least 40 seconds, more preferably at least 50 seconds, most preferably at least about 60 seconds, and preferably over a period of at most 120 seconds, preferably at most 90 seconds, more preferably at most 80 seconds, particularly at most 70 seconds, most preferably at most about 60 seconds.

A 76^{th} embodiment of the present invention is the process of any one of the 73^{rd} to 75^{th} embodiments, wherein the textile material is a fabric of at least 350 grams per m² and curing takes place at the curing temperature as defined in the 60^{th} embodiment over a period of at least 45 seconds, preferably at least 60 seconds, more preferably at least 75 seconds, most preferably at least about 90 seconds, and preferably over a period of at most 180 seconds, preferably at most 160 seconds, more preferably at most 140 seconds, particularly at most 120 seconds, most preferably at most about 90 seconds.

A 77^{th} embodiment of the present invention is the process of any one of the 73^{rd} to 76^{th} embodiments, wherein the textile material is a fabric of at least 500 grams per m² and curing takes place at the curing temperature as defined in the 60^{th} embodiment over a period of at least 60 seconds, preferably at least 75 seconds, more preferably at least 90 seconds, most preferably at least about 120 seconds, and preferably over a period of at most 240 seconds, preferably at most 210 seconds, more preferably at most 180 seconds, particularly at most 150 seconds, most preferably at most about 120 seconds. A 78^{th} embodiment of the present invention is the process of any one of the 73^{rd} to 77^{th} embodiments, wherein curing immediately follows drying of the textile material without the textile material substantially cooling down between drying of the textile material and curing.

A 79^{th} embodiment of the present invention is the process of the 78^{th} embodiment, wherein the textile material is a fabric and drying of the textile material and curing are performed over a period of together at least 45 seconds, preferably at least 50 seconds, more preferably at least 55 seconds, most preferably at least about 60 seconds, per 100 grams of fabric weight per square meter.

An 80^{th} embodiment of the present invention is the process of the 78^{th} or 79^{th} embodiment, wherein the textile material is a fabric and drying of the textile material and curing are performed over a period of together at most 75 seconds, preferably at most 70 seconds, more preferably at most 65 seconds, most preferably at most about 60 seconds, per 100 grams of fabric weight per square meter.

An 81^{st} embodiment of the present invention is the process of any of the 60^{th} to 80^{th} embodiments, wherein where the antimicrobial and/or second liquor application process is an exhaustion process, the liquor has a temperature of at least 45 °C, preferably of at least 50 °C, more preferably of at least 60 °C, particularly of at least 70 °C, and most preferably of at least about 80 °C.

An 82^{nd} embodiment of the present invention is the process of any of the 60^{th} to 72^{nd} embodiments, wherein where the antimicrobial and/or hydrophilic/stain release liquor application process is an exhaustion process, the liquor has a temperature below boiling temperature, preferably of at most 95 °C, more preferably of at most 90 °C, particularly of at most 85 °C, and most preferably of at most about 80 °C.

An 83^{rd} embodiment of the present invention is the process of any of the 60^{th} to 82^{nd} embodiments, wherein the exhaustion process time is at most 90 min, preferably at most 80 min, more preferably at most 70 min, and most preferably at most about 60 min.

An 84^{th} embodiment of the present invention is the process of any of the 60^{th} to 83^{rd} embodiments, wherein the exhaustion process time is at least 45 min, preferably at least 50 min, more preferably at least 55 min, and most preferably at least about 60 min.

An 85^{th} embodiment of the present invention is the process of any of the 60^{th} to 84^{th} embodiments, wherein in a padding process of the antimicrobial and/or hydrophilic/stain release liquor application process, the liquor pickup rate is at least 30%, preferably at least 40%, more preferably at least 50%, particularly at least 60% or at least 80%, and most preferably at least about 100%, and/or at most 140%, preferably of at most 130%, more preferably of at most 120%, particularly at most 110%, and most preferably of at most about 100%

An 86^{th} embodiment of the present invention is the process of any of the 60^{th} to 85^{th} embodiments, wherein in a padding process of the antimicrobial and/or hydrophilic/stain release liquor application process, the textile material passes through a multiple trough padding mangle, or a continuous dyeing or bleaching range.

An 87^{th} embodiment of the present invention is the process of any of the 60^{th} to 86^{th} embodiments, wherein the one or more antimicrobial agents are selected and/or applied as defined in any one of the 5^{th} to 14^{th} embodiments.

An 88^{th} embodiment of the present invention is the process of any of the 60^{th} to 87^{th} embodiments, wherein the liquor of the hydrophilic/stain release liquor application process contains metal, preferably copper, zinc, or silver, more preferably silver cations.

An 89^{th} embodiment of the present invention is the process of the 88^{th} embodiment, wherein the liquor of the hydrophilic/stain release liquor application process contains such amounts of metal that in the hydrophilic/stain release liquor application process, the metal is applied to the textile material in an amount of at most 0.05%, preferably at most 0.01%, more preferably at most 0.005%, even more preferably at most 0.003%, and most preferably at most about 0.0017% on weight fabric of the textile material.

A 90^{th} embodiment of the present invention is the process of any of the 88^{th} or 89^{th} embodiments, wherein the liquor of the hydrophilic/stain release liquor application process contains such amounts of metal that in the hydrophilic/stain release liquor application process, the metal is applied to the textile material in an amount of at least 0.0001%, preferably at least 0.0005%, more preferably at least 0.001%, and most preferably at least about 0.0017% on weight fabric of the textile material.

A 91^{st} embodiment of the present invention is the process of any of the 60^{th} to 90^{th} embodiments, wherein the antimicrobial agents are adhered to the textile material with a total weight as defined in the 15^{th} or 16^{th} embodiment, and/or an individual weight as defined for the respective antimicrobial agents in any one of the 17^{th} to 26^{th} embodiments.

A 92^{nd} embodiment of the present invention is the process of any of the 60^{th} to 86^{th} embodiments, wherein the one or more hydrophilic agents are selected and/or applied as defined in the 28^{th} embodiment.

A 93^{rd} embodiment of the present invention is the process of any of the 60^{th} to 92^{nd} embodiments, wherein the one or more stain release agents are selected and/or applied as defined in the 29^{th} embodiment.

A 94^{th} embodiment of the present invention is the process of any of the 60^{th} to 93^{rd} embodiments, wherein the hydrophilic and/or stain release agents are adhered to the textile material with a total weight as defined in any one of the 32^{nd} to 35^{th} embodiments.

A 95^{th} embodiment of the present invention is the process of any of the 60^{th} to 94^{th} embodiments, wherein the (starting) textile material is a material as defined in any one of the 51^{st} to 59^{th} embodiments.

A 96^{th} embodiment of the present invention is the process of any of the 60^{th} to 95^{th} embodiments, wherein the textile material obtained by the process is a textile material according to any one of the 1^{st} to 59^{th} embodiments.

A 97^{th} embodiment of the present invention is a textile material obtainable according to the process of any one of the 60^{th} to 96^{th} embodiments.

A 98^{th} embodiment of the present invention is a multi-layer filter material comprising
(a) at least one layer of the textile material of any of the 1^{st} to 59^{th} embodiments, or the 97^{th} embodiment, and
(b) at least one layer of particulate filter material.
A 99^{th} embodiment of the present invention is the multi-layer filter material of the 98^{th} embodiment, wherein the particulate filter material is an at least N_{90/}R_{90/}P₉₀-rated, preferably an at least N_{95/}R_{95/}P₉₅-rated, and most preferably an at least N₉₉/R₉₉/P₉₉-rated non-woven fabric.

A 100^{th} embodiment of the present invention is the multi-layer filter material of the 98^{th} or 99^{th} embodiments, wherein two layers of the textile material are used as outer layers to sandwich the at least one layer of particulate filter.

A 101^{st} embodiment of the present invention is the multi-layer filter material of any of the 98^{th} to 100^{th} embodiments, wherein the textile material layers are adhered to the at least one layer of particulate filter by means of ultrasonic welding.

A 102^{nd} embodiment of the present invention is the multi-layer filter material of any of the 98^{th} to 101^{st} embodiments, wherein the multi-layer filter material exhibits a filter efficiency of at least 96%, preferably of at least 97%, most preferably 98% in accordance with test method IS 9473-2002, measured for the passing percentage of a sodium chloride aerosol (method option A-4.1).

A 103^{rd} embodiment of the present invention is the multi-layer filter material of any of the 98^{th} to ₁₀₂n^{d} embodiments, wherein the multi-layer filter material comprises at least two layers, more preferably at least three layers of particulate filter material as defined in the 99^{th} embodiment, preferably the particulate filter material being at least N95/R95/P95-rated.

A 104^{th} embodiment of the present invention is the multi-layer filter material of any of the 1^{st} to 59^{th} embodiments, or the 97^{th} embodiment, or any one of the 98^{th} to 103^{rd} embodiments, wherein at least 95%, preferably at least 97%, more preferably at least 99% of the starting textile material consists of a synthetic fiber, such as polyamide (nylon) or preferably polyester.

A 105^{th} embodiment of the present invention is an air filter comprising a multi-layer filter material as defined in any one of the 98^{th} to 104^{th} embodiments.

A 106^{th} embodiment of the present invention is the air filter of the 105^{th} embodiment, wherein the air filter exhibits a bacterial filtration efficiency (BFE) of at least 99.9% (3 log), preferably of at least 99.99% (4 log), most preferably of at least 99.999% (5 log) as measured in accordance with test method ASTM F2101-2014 (test organism: Staphylococcus aureus (ATCC 6538); inoculum size: 6.5 x 106 CFU/ml; flow rate: 1 Cu. Ft./min; aerosol particle size: 3 ± 0.5 µm; distance: 15 cm).
A 107^{th} embodiment of the present invention is the air filter of any of the 105^{th} or 106^{th} embodiment, wherein the air filter exhibits a filter efficiency of at least 96%, preferably of at least 97%, most preferably 98% in accordance with test method IS 9473-2002, measured for the passing percentage of a sodium chloride aerosol (method option A-4.1).

A 108^{th} embodiment of the present invention is the air filter of any of the 105^{th} to 107^{th} embodiments, wherein the claimed bacterial filtration efficiency (BFE) is achieved even after at least 25 laundry washes in a laundry washing machine at 85 ±15 °C for 10-15 minutes, preferably using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

A 109^{th} embodiment of the present invention is the air filter of any of the 105^{th} to 108^{th} embodiments, wherein the claimed filter efficiency is achieved even after at least 25 laundry washes in a laundry washing machine at 85 ±15 °C for 10-15 minutes, preferably using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

A 110^{th} embodiment of the present invention is the air filter of any of the 105^{th} to 109^{th} embodiments, wherein the textile material and the particulate filter material substantially have the same dimensions.

A 111^{th} embodiment of the present invention is the air filter of any one of the 105^{th} to 110^{th} embodiment, wherein the layers of the multi-layer filter material are attached to each other by means of ultrasonic welding.

A 112^{th} embodiment of the present invention is the air filter of any of the 105^{th} to 111^{th} embodiments, wherein the air filter is a face mask.

A 113^{th} embodiment of the present invention is the face mask of the 112^{th} embodiment, wherein the face mask has the shape of a pad.

A 114^{th} embodiment of the present invention is the face mask of any of the 112^{th} or 113^{th} embodiment, wherein the pad has a width in the range of from 5 cm to 15 cm.

A 115^{th} embodiment of the present invention is the face mask of any one of the 112^{th} or 114^{th} embodiments, wherein the pad has a length in the range of 10 cm to 25 cm.

A 116^{th} embodiment of the present invention is the face mask of any one of the 112^{th} to 115^{th} embodiments, wherein at least two expansion pleats are integrated to balloon over nose and face.

A 17^{th} embodiment of the present invention is the face mask of any one of the 112^{th} to 116^{th} embodiments, wherein a nose clip is integrated to fix the face mask to the nose.

A 118^{th} embodiment of the present invention is the face mask of any one of the 112^{th} to 17^{th} embodiments, wherein the pad comprises fixing means to attach the pad to the face, such as ear loops or straps to be fixed around the head and/or neck.

A 119^{th} embodiment of the present invention is the face mask of any one of the 112^{th} to 118^{th} embodiments, wherein the nose clip and/or the fixing means are adhered to the multi-layer filter material by means of ultrasonic welding.

A 120^{th} embodiment of the present invention is the use of the multi-layer filter material as defined in any one of the 98^{th} to 104^{th} embodiments as a washable air filter.

A 121^{st} embodiment of the present invention is the use of the washable air filter of the 120^{th} embodiment for use as a filter in air conditioners and other air-filtering and/or circulating systems, such as in in building or vehicle ventilation systems.

A 122^{nd} embodiment of the present invention is the use of the washable air filter of the 120^{th} embodiment for use as a filter in fresh air-inlets or exhaust air-outlets.

A 123^{rd} embodiment of the present invention is the use of the washable air filter of the 120^{th} embodiment for use as a filter to filter air that is stored in gas bottles, such as for scuba diving, and the like.

A 124^{th} embodiment of the present invention is the use of the multi-layer filter material as defined in any one of the 98^{th} to 104^{th} embodiments as a washable face mask.

A 125^{th} embodiment of the present invention is the use of the washable face mask of the 124^{th} embodiment as respiratory protective face mask against airborne environmental pollution, such as fine dust and/or pathogens that are transmitted via droplet infection.

A 126^{th} embodiment of the present invention is the use of the washable face mask of any of the 124^{th} or 125^{th} embodiment as respiratory protective face mask against airborne industrial pollution, such as industrial dust and/or contaminating pathogens that are transmitted via droplet infection.

### DETAILED DESCRIPTION OF PREFERRED EMBODIMENTS

In the following, preferred embodiments of the invention are described with reference to the figures, in which:
- Fig. 1: is a flowchart illustrating a process of finishing a textile material according to one embodiment of the invention;
- Fig. 2: is a flowchart illustrating a process of finishing a textile material according to a preferred embodiment of the invention;
- Fig. 3: specifies the antimicrobial performance of one single antimicrobial agent applied to a polyester textile material according to experimental examples of the invention;
- Fig. 4: specifies the antimicrobial performance of two antimicrobial agents applied to a polyester textile material according to experimental examples of the invention;
- Fig. 5: specifies the antimicrobial performance of three antimicrobial agents applied to a polyester textile material according to experimental examples of the invention;
- Fig. 6: specifies the performance of particle penetration and breathing resistance of face masks according to experimental examples of the invention respectively having one to four layers of N₉₅-rated particulate filtering material;
- Fig. 7: specifies the performance of particle penetration and breathing resistance of face masks according to experimental examples of the invention respectively having one to four layers of N₉₀-rated particulate filtering material;
- Fig. 8: specifies the performance of particle penetration and breathing resistance of face masks according to experimental examples of the invention respectively having one to four layers of N₉₉-rated particulate filtering material;
- Fig. 9: specifies the antimicrobial performance of the face mask according to the Working Example
- Fig. 10: specifies the composition of and the manufacturing process for obtaining the face mask according to the Working Example
- Fig. 11: specifies the wash performance of the face mask according to the Working Example
- Fig. 12: specifies the bacterial filtration efficiency of the Working Example in comparison to the competitor products Vogmask N₉₉ and 3M 8210 [N95]

### Antimicrobial agents

### Preferred antimicrobial agents

A great variety of antimicrobial agents can be fixed to a textile by using the process of the invention described below. The antimicrobial agents are preferably non-ionic or cationic, but not anionic. The inventors found that anionic compounds do not bind well to textiles and can easily be removed, e.g. by salts. Cationic (acid) agents are believed to attack the textile and therefore attach to it. Nanoparticles or antimicrobials in the form of nanoparticles are not preferred. Rather, the one or more antimicrobial agents have a preferred particle size, in all dimensions (length, width, height), of at least 250 nanometers, preferably at least 500 nanometers, more preferably at least 750 nanometers, and most preferably at least 1,000 nanometers.

According to the preferred embodiments of the invention, an antimicrobial agent is selected from quaternary ammonium organosilane compounds, metal, polyglucosamine (chitosan), azole-based compounds, and polyhexamethylene biguanide (PHMB).

As will be described for the preferred antimicrobial agents in detail below, the antimicrobial agents are bound to the textile material preferably either directly, in particular if the agent is a quaternary ammonium organosilane compound, polyglucosamine, a silver, copper, or zinc cation, which can be trapped in an inorganic or organic matrix, or PHMB, or via cross linking, in particular if the agent is an azole-based compound. The use of cyclodextrin and/or an inclusion complex, in particular an inclusion complex of fiber-reactive cyclodextrin derivatives and antimicrobial agents is not preferred for binding the antimicrobial agents, in particular because cyclodextrin is prohibitively expensive for most applications.

Suitable quaternary ammonium organosilane compounds have the formula 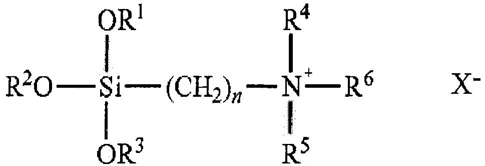 wherein the radicals have, independently of each other, the following meanings: R¹, R₂, and R³ are a C₁-C₁₂-alkyl group, in particular a C₁-C₆-alkyl group, preferably a methyl group; R⁴, and R⁵ are a C₁-C₁₈-alkyl group, a C₁-C₁₈-hydroxyalkyl group, a C₃-C₇-cycloalkyl group, a phenyl group, or a C₇-C₁₀-aralkyl group, in particular a C₁-C₁₈-alkyl group, preferably a methyl group; R⁶ is a C₁-C₁₈-alkyl group, in particular a C₈-C₁₈-alkyl group; X- is the counterion and an anion, for example, chloride, bromide, fluoride, iodide, acetate, or a sulfonate group, preferably X- is chloride or bromide; and n is an integer of 1 to 6, in particular an integer of 1 to 4, preferably 3. The term "alkyl group" as used herein means a branched or unbranched alkyl group.

Quaternary ammonium organosilane compounds are known in the art and commercially available. Such compounds possess specific functional groups which enable their bonding to functional groups of the textile material. Under the reaction conditions disclosed herein, the quaternary ammonium organosilane compounds are bonded to the textile material via a covalent bond between the organosilane moiety and functional groups of the textile. Furthermore, organosilane moieties polymerize with each other, resulting in -O-Si-O- bonds. A possible reaction mechanism of the ammonium organosilane with a textile material having hydroxyl groups is shown hereinafter.

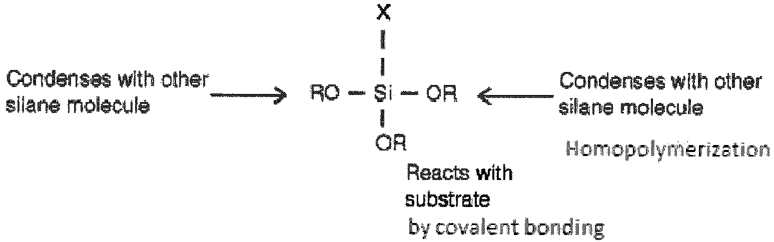

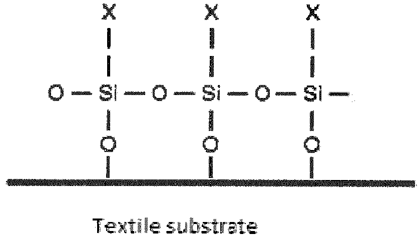

A possible reaction mechanism of the ammonium organosilane with silk having peptide groups (-CO-NH-) is shown hereinafter.

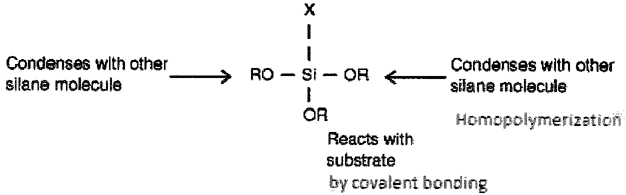

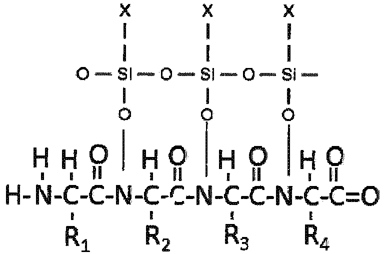

The quaternary ammonium organosilane compound can comprise dimethyloctadecyl[3-(trimethoxysilyl)propyl] ammonium chloride or dimethyltetradecyl[3-(trimethoxysilyl)propyl] ammonium chloride, most preferably dimethyloctadecyl[3-(trimethoxysilyl)propyl]ammonium chloride. The structure of dimethyloctadecyl[3-(trimethoxysilyl)propyl]ammonium is as follows (shown without counterion), wherein further the function of the silane moiety and the ammonium moiety are indicated:

### Organosilane quaternary amine

### Molecular Structure:

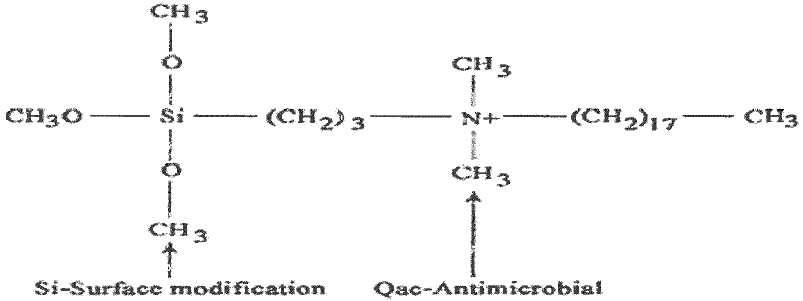

Dimethyloctadecyl[3-(trimethoxysilyl)propyl] ammonium chloride is available on the market, e.g. in AEM 5772 (manufactured by Aegis, USA). Dimethyltetradecyl[3-(trimethoxysilyl)propyl] ammonium chloride is available on the market, e.g. in Sanitized T 99-19 (manufactured by Sanitized AG, Switzerland). Other suitable ammonium silane compounds are described, e.g., in patent applications US 2011/0271873 A1 and US 2006/0193816 A1, and in US patent 8,906,115.

The metal may be copper, zinc, or preferably silver, more preferably silver cations. Tests made by the inventor, whose results are not reproduced herein, showed that copper nitrate and zinc nitrate have similar antimicrobial properties as silver cations, and they can be adhered to textile materials in similar ways. However, the antimicrobial performance of silver is considerably higher than that of zinc, and even much higher than that of copper. Furthermore, treating textile materials with copper or zinc tends to lead more easily to a change of the shade of textile than a treatment with silver.

In particular embodiments, the silver cations are trapped in an inorganic or organic matrix. Preferably, the inorganic matrix is an aluminosilicate. Preferably, the organic matrix is a polymeric matrix. Such silver-containing microbial agents are known in the art and available on the market.

A silver cation in form of its acrylate salt is shown hereinafter.

### Silver acrylate salt

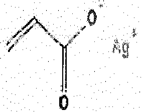

In an exemplary embodiment of the invention, the aluminosilicate is a sodium-poly(sialate-disiloxo) compound. Examples of an aluminosilicate and sialate structures as well as how bonding to a textile material can occur under the reaction conditions disclosed herein, are shown hereinafter.

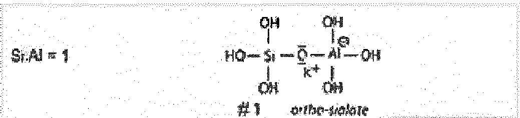

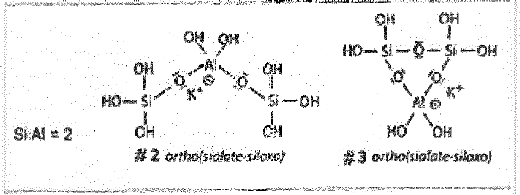

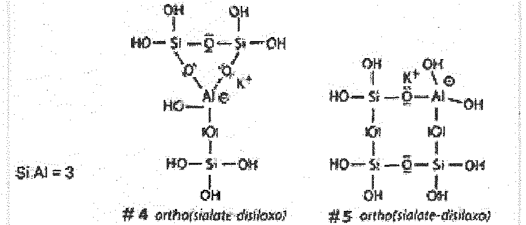

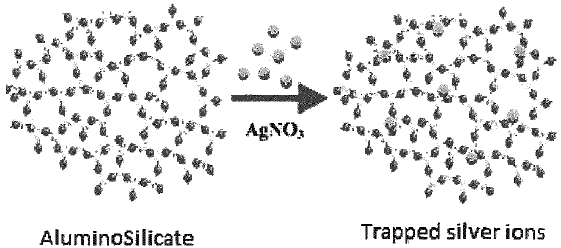

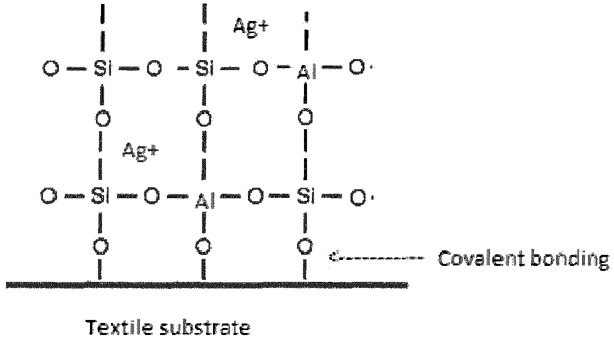

In an exemplary embodiment of the invention, the polymeric matrix into which silver cations are trapped is an acrylic polymer. Such silver-containing agents are known in the art and available on the market, e.g. in SilvaDur AQ Antimicrobial of Rohm and Haas, Switzerland, which contains acrylic polymer(s), silver nitrate, nitric acid and water. In another exemplary embodiment of the invention, the silver cations are trapped in a polymeric matrix. Such silver-containing agents are known in the art and available on the market, e.g. in SILVADUR™ 930 Antimicrobial of Dow Chemical Company, USA, which contains polymer(s), silver cations, ammonia, ethanol and water.

Polyglucosamine (chitosan) has a structure as shown hereinafter, wherein n indicates the number of monomer units as known in the art:

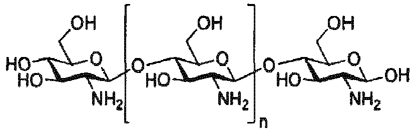

Under the reaction conditions disclosed herein, chitosan can react with -NH groups of silk resulting in covalent bonds as shown below.

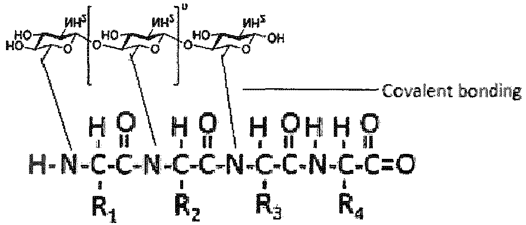

Under the reaction conditions disclosed herein, chitosan can react with functional groups of cellulosic materials resulting in covalent bonds as shown below.

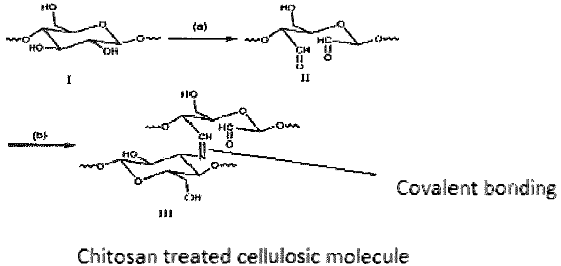

Chitosan is known in the art and commercially available, e.g. in Goyenchem-102 of Go Yen Chemical, Taiwan. It is particularly effective for killing viruses. It cannot easily be bound to synthetic textile materials, in particular it is difficult to bind it in a wash-durable or even substantially non-leaching manner.

Polyhexamethylene biguanide (PHMB) has a structure as shown hereinafter, wherein n indicates the number of monomer units as known in the art.

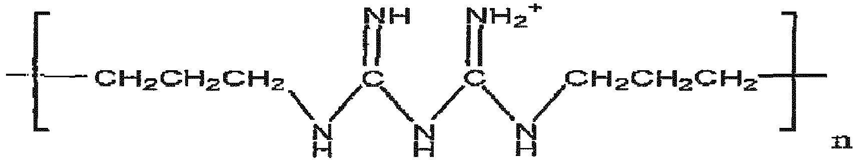

Under the reaction conditions disclosed herein, polyhexamethylene biguanide can react with hydroxyl groups of cellulose to form covalent bonds as shown hereinafter.

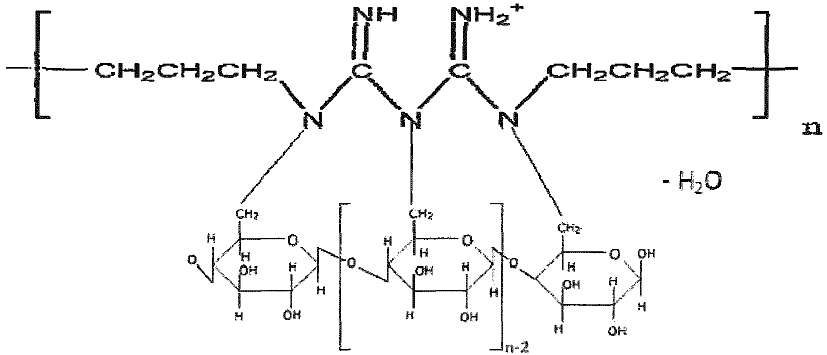

Under the reaction conditions disclosed herein, PHMB can react with carbonyl groups of silk fiber to form covalent bonds as shown hereinafter.

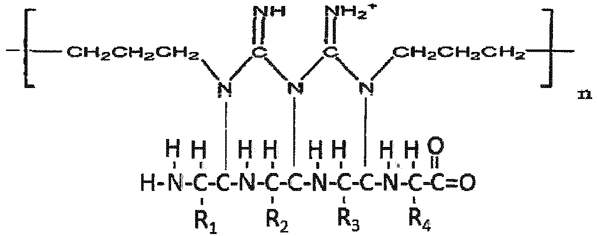

PHMB is known in the art and commercially available, e.g. in Texguard 20 of Swissol Specialties Pvt. Ltd, India. It cannot easily be bound to synthetic textile materials, in particular it is difficult to bind it in a wash-durable or even substantially non-leaching manner.

The azole-based compound can be, e.g., thiabendazole, carbendazim or preferably a triazole-based compound. Tests made by the inventor, whose results are not reproduced herein, showed that thiabendazole has similar antimicrobial properties as the triazole-based compounds, and it can be adhered to textile materials in similar ways. However, the antimicrobial performance of triazole-based compounds is considerably higher than that of thiabendazole.

The triazole-based compound is preferably propiconazole. Propiconazole has a structure as shown hereinafter.

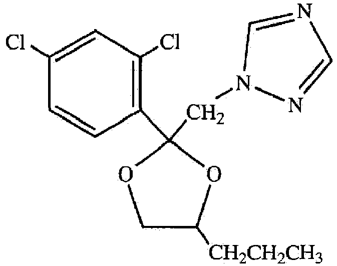

Propiconazole is known in the art and commercially available, e.g. in Biogard PPZ 250 of Beyond Surface Technologies, Switzerland. Propiconazole can be bound to the textile material using a crosslinking agent, in particular a preferably blocked isocyanate compound, which results in urethane bonds, or an acrylate based-product. When using propiconazole, it is preferred to use a crosslinking agent in the liquor, in particular an exhaust liquor. It is even more preferred that the formulation of propiconazole contains the cross linking agent or the cross-linking agent is part of the propiconazole formulation. Furthermore, it is preferred to use propiconazole together with an emulsifier. Propiconazole is particularly effective for killing fungi.

### Combinations of several antimicrobial agents

In preferred embodiments of the invention, at least two, at least three, at least four, or all five of the antimicrobial agents selected from the above-mentioned group consisting of quaternary ammonium organosilane compounds, metal, chitosan, azole-based compounds, and PHMB are adhered to a textile material.

The use of a combination of several antimicrobial agents has the following advantages over the use of a single agent:
First of all, different agents have different antimicrobial effects. Some may work better against bacteria, others against viruses, and again others against fungi. Adding a variety of agents increases the spectrum of microbes which can be killed when they come in contact with the antimicrobial textile.

Secondly, the use of a variety of agents can lead to significantly higher killing rates, even for the same organism. It is believed that the higher killing rates are due to synergistic effects between the different agents. The different agents may work synergistically together due to their different killing mechanisms.

Thirdly, the use of different agents may allow binding a higher total amount of agents to the textile. For each of the different agents, there is an inherent limit on the quantity of the agent that can be adhered to the textile in a non-leaching or substantially non-leaching manner. However, even if a textile material has been saturated with and for one agent, there may still be "space" for another agent.

Forth, the use of several agents allows reducing the leaching rates per agent. It is less the total amount of leached substances that will determine the threat to health and environment, but rather the amount per substance. Thus, although the total amount of leached substances may be the same, the leaching values per agent are lower, which is highly beneficial.

Fifth, inherent undesired effects of a substance can be reduced or even counterbalanced by the use of several agents. For example, organosilane is hydrophobic by nature, which is an undesired property for many applications of textiles. For such applications, the concentration of organosilane should be kept at a minimum.

Sixth, some of the preferred agents of the present invention are more expensive than others, e.g. silver cations and chitosan. Reducing the concentrations of these agents and complementing them by other agents allows achieving the antimicrobial performance at substantially lower costs.

These advantages could be shown by the inventor in a series of experiments, some of which are presented in International patent application PCT/EP2016/054245 by the same applicant, and some of which will be presented below.

It is one merit of the invention to have recognized the advantages of using several antimicrobial agents in combination. It is another merit of the invention to have identified several highly effective antimicrobial agents which can be bound to a textile material together, which are relatively inexpensive, and low in toxicity. It is a further merit of the invention to have identified a process, which will be described below, by which many different agents can be applied to a textile material in one and the same liquor application process, be it in one or more application cycles, in a wash-durable or even substantially non-leaching manner. According to the preferred embodiments, wash-durable means that any agents that achieve a property of the textile material are essentially permanently adhered to a textile material, and the property is present even after at least 25 laundry washes in a laundry washing machine at 85±15 °C for 10-15 minutes using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

In some embodiments of the invention, at least one, preferably at least two, more preferably at least three, and most preferably all four selected from the group consisting of metal, azole-based compounds, chitosan, and quaternary ammonium organosilane are adhered to a textile material. This is the same group as the group of five mentioned above but without PHMB. Such a combination is particularly suitable where a synthetic textile material shall be finished. PHMB cannot be easily bound to synthetic textile materials, in particular not in a wash-durable or even substantially non-leaching manner.

In some embodiments of the invention, at least one, preferably at least two, more preferably all three selected from the group consisting of metal, azole-based compounds, and quaternary ammonium organosilane compounds are adhered to a textile material. This is the same group as the group of five mentioned above but without chitosan and PHMB. Such a combination is particularly suitable where the starting textile is substantially made of a synthetic material and comprises no significant amounts of cellulosic material because chitosan and PHMB cannot easily be bound to synthetic textile materials, in particular not in a wash-durable or even substantially non-leaching manner.

In some embodiments of the invention, at azole-based compounds and at least one, preferably at least two, or all least three, selected from the group consisting of metal, PHMB and quaternary ammonium organosilane compounds are adhered to a textile material. The azole-based compounds, in particular propiconazole, are highly efficient against fungi, whereas the other three agents are highly efficient against bacteria. Therefore, azole-based compounds and at least one of the other three agents complement each other well, for manufacturing textiles that are effective against both bacteria and fungi.

### Amounts of antimicrobial agents adhered to the textile material

The ideal percentage of the chemicals present on the textile material has been elaborated in an extensive research and development effort and is in part based on the work discussed in International patent application PCT/EP2016/054245 by the same applicant.

Preferably, the total amount of the one or more antimicrobial agents adhered to the textile material is at most 4.0%, preferably at most 3.0%, more preferably at most 2.5%, and most preferably at most about 2.0% on weight fabric of the textile material. These amounts represent the maximum amount of antimicrobial agents the textile can take in, and if higher amounts are applied, leaching of the agents increases significantly. Furthermore, the total amount of the one or more antimicrobial agents adhered to the textile material is preferably at least 0.05%, preferably at least 0.1%, more preferably at least 0.2%, even more preferably at least 0.4%, particularly at least 0.6%, and most preferably at least about 0.9% on weight fabric of the textile material, to ensure high antimicrobial efficiency.

In preferred embodiments, PHMB is adhered to the textile material in an amount of at most 1.0%, preferably at most 0.8%, more preferably at most 0.6%, and most preferably at most about 0.4% on weight fabric of the textile material. Furthermore, the PHMB is adhered to the textile material in an amount of preferably at least 0.05%, more preferably at least 0.1%, even more preferably at least 0.2%, in particular at least 0.3%, and most preferably at least about 0.4% on weight fabric of the textile material.

In preferred embodiments, metal is adhered to the textile material in an amount of at most 0.14%, preferably at most 0.12%, more preferably at most 0.10%, even more preferably at most 0.08%, and most preferably at most about 0.06% on weight fabric of the textile material. Furthermore, the metal is adhered to the textile material in an amount of preferably at least 0.0005%, more preferably at least 0.001%, even more preferably at least 0.005%, and most preferably at least about 0.002% on weight fabric of the textile material.

In preferred embodiments, azole-based compounds are adhered to the textile material in an amount of at most 1.0%, preferably at most 0.8%, more preferably at most 0.6%, and most preferably at most about 0.4% on weight fabric of the textile material. Furthermore, the azole-based compounds are adhered to the textile material in an amount of preferably at least 0.02%, more preferably at least 0.05%, even more preferably at least 0.10%, and most preferably at least about 0.20% on weight fabric of the textile material.

In preferred embodiments, chitosan is adhered to the textile material in an amount of at most 1.5%, preferably at most 1.0%, more preferably at most 0.7%, in particular at most 0.5%, and most preferably at most about 0.3% on weight fabric of the textile material. Furthermore, chitosan is adhered to the textile material in an amount of preferably at least 0.05%, more preferably at least 0.1%, even more preferably at least 0.2%, and most preferably at least 0.3% on weight fabric of the textile material.

In preferred embodiments, quaternary ammonium organosilane compounds are adhered to the textile material in an amount of at most 0.8%, preferably at most 0.6%, more preferably at most 0.3%, in particular at most 0.15%, and most preferably at most about 0.08% on weight fabric of the textile material. Furthermore, the quaternary ammonium organosilane compounds are adhered to the textile material in an amount of preferably at least 0.01%, preferably at least 0.02%, more preferably at least 0.04%, and most preferably at least about 0.06% on weight fabric of the textile material. The inventor found that if quaternary ammonium organosilane is applied to synthetic textile materials such as polyester or polyamide, amounts of more than 0.15% decrease the possibility to adhere hydrophilic and/or stain release agents in a subsequent processing step in a wash-durable manner. For cellulosic textiles such as cotton or viscose, the ability to bond hydrophilic and/or stain release agents in a wash-durable manner decreases even at lower amounts. Therefore, the use of quaternary ammonium organosilane is not preferred for cellulosic textiles where hydrophilic and/or stain release agents are to be adhered to the textile as well.

### Antimicrobial efficiency

The one or more antimicrobial agents adhered to the textile material preferably increase the reduction value of the textile material compared to the starting textile material, and/or the treated textile material preferably exhibits a reduction value of Escherichia coli ATCC 25922 and/or Staphylococcus aureus ATCC 6538 and/or Pseudomonas aeroginosa ATCC 15442 and/or Salmonella enterica ATCC 10708 and/or Candida albicans ATCC 10231 and/or Aspergillus niger 16404 measured in accordance with AATCC test method 100-2012 by/of at least 90% (1 log), preferably at least 99% (2 log), more preferably at least 99.9% (3 log) within 10 minutes of contact time and/or of at least 99% (2 log), preferably at least 99.9% (3 log), more preferably at least 99.99% (4 log) within 1 hour of contact time and/or of at least 99.% (2 log), preferably at least 99.9% (3 log), more preferably at least 99.99% (4 log), most preferably at least 99.999% (5 log) within 24 hours of contact time.

Furthermore, a textile material according to preferred embodiments of the invention exhibits after 25 laundry washes a reduction value of Escherichia coli ATCC 25922 and/or Staphylococcus aureus ATCC 6538 and/or Pseudomonas aeroginosa ATCC 15442 and/or Salmonella enterica ATCC 10708 and/or Candida albicans ATCC 10231 and/or Aspergillus niger 16404 of at least 99%, preferably at least 99.9%.

### Hydrophilic Agents/Stain Release Agents

A great variety of hydrophilic agents/stain release agents can be fixed to a textile by using the process of the invention described below. Like the preferred antimicrobial agents, the hydrophilic agents/stain release agents are preferably non-ionic or cationic, but not anionic.

According to preferred embodiments of the invention, the one or more hydrophilic agents adhered to the textile material comprise at least one selected from the group consisting of carboxylic acid esters, polyester ether copolymers, starch based emulsions, fatty alcohol ethoxylates, and organosilane terpolymers, preferably fatty alcohol ethoxylates or organosilane terpolymers, most preferably organosilane terpolymers.

According to preferred embodiments of the invention, the one or more stain release agents adhered to the textile material comprise at least one selected from the group consisting of fatty alcohol ethoxylates and organosilane terpolymers, preferably organosilane terpolymers. In this case, the one or more or all of the stain release agents can convey hydrophilic properties to the textile as well, which for many applications like surface sanitizers or cleaning wipes is particularly advantageous.

Organosilane terpolymer is known in the art and commercially available, e.g. in Hydrosil 8900, of Britacel Silicones Ltd., India. It is based on modified silicone and applicable for both natural (cellulosic) as well as synthetic textiles.

Fatty alcohol ethoxylates are known in the art and commercially available, e.g. in Hydroperm of Archroma, which contains ultra-fine polyester granules and is most suitable for cellulosic textiles, or in Permalose of Croda Chemicals, India, which contains ultra-fine polyurethane granules and is most suitable for synthetic textiles like polyester.

Both organosilane terpolymer and fatty alcohol ethoxylates are in preferred embodiments covalently bonded, or strongly fixed, optionally by using an inbuilt binder, to the textile material such that they can withstand numerous washes. In preferred embodiments, the hydrophilic/stain release agents, in particular organosilane terpolymer and/or fatty alcohol ethoxylates are bonded without the use of a crosslinker, in particular without the use of isocyanate, more particularly blocked isocyanate.

It is one merit of the invention to have recognized that the comfort of wearing face masks increases when the textile material constituting the outer layers of the multi-layer filter material from which the face mask is made is hydrophilic. It is another merit of the invention to have recognized that the washability of such textiles can be increased by improving the stain release properties of the textiles. It is another merit of the invention to have identified several highly effective hydrophilic and stain release agents that achieve these properties. It is a further merit of the invention to have identified a process by which hydrophilic/stain release properties and in particular both antimicrobial and hydrophilic/stain release properties can be conveyed to a textile material, be it in one or more liquor application cycles, in a wash-durable or even substantially non-leaching manner. Finally, it is a merit of the invention to have identified combinations and amounts of antimicrobial agents and hydrophilic/stain release agents to be adhered to a textile material such that the hydrophilic/stain release agents do not prevent a satisfactory antimicrobial efficiency of the textile material, and the antimicrobial agents do not prevent satisfactory hydrophilic/stain release properties of the textile material.

### Amounts of hydrophilic agents/stain release agents adhered to the textile material

In preferred embodiments, the hydrophilic and/or stain release agents are adhered to the textile material in an amount of together at most 6%, preferably at most 5%, more preferably at most 4%, even more preferably at most 3%, in particular at most 2.5%, and most preferably at most about 1.75% on weight fabric of the textile material, and/or in an amount of together at least 0.25%, preferably at least 0.5%, more preferably at least 0.75%, particularly at least 1% and most preferably at least about 1.25% on weight fabric of the textile material.

Organosilane terpolymers are preferably adhered to the textile material in an amount of at least 0.1%, preferably at least 0.2%, more preferably at least 0.5%, particularly at least 1.0%, and most preferably at least about 1.5% on weight fabric of the textile material. Fatty alcohol ethoxylates are preferably adhered to the textile material in an amount of at most 4%, preferably at most 3%, more preferably at most 2%, even more preferably at most 1.5%, and most preferably at most about 1.0% on weight fabric of the textile material. The inventor found that when more than 1.5% o.w.f. of organosilane terpolymers or more than 1.0% o.w.f. of fatty alcohol ethoxylates are adhered to a textile material, the efficiency of any antimicrobial agents adhered to the textile starts to decrease.

### Efficiency of the hydrophilic agents/stain release agents

In preferred embodiments of the invention, the one or more hydrophilic agents adhered to the textile material reduce the water absorbency time of the starting textile material by at least 20%, preferably at least 40%, more preferably at least 60%, and most preferably at least 80%, preferably when measured in accordance with AATCC test method 79-2014 (Option A). The treated textile material preferably exhibits a water absorbency time of at most 5 seconds, preferably at most 3 seconds, more preferably at most 2 seconds, and most preferably at most 1 second, preferably when measured in accordance with AATCC test method 79-2014 (Option A).

In some embodiments, the one or more hydrophilic agents adhered to the textile material preferably do not increase the water repellency of the starting textile material, measured in accordance with AATCC test method 22-2014. The treated textile material preferably exhibits a water repellency rating of at most 50, preferably o, measured in accordance with AATCC test method 22-2014.

In some embodiments, the one or more hydrophilic agents adhered to the textile material increase the vertical wicking rate of the starting textile material by at least a 10%, preferably at least 20%, and most preferably at least 30%, when tested according to AATCC test method 197-2013. The treated textile material preferably exhibits a vertical wicking rate of at least 0.15 mm/sec, preferably at least 0.18 mm/sec, more preferably at least 0.20 mm/sec, and most preferably at least 0.23 mm/sec, when tested according to AATCC test method 197-2013. The vertical wicking rate is the speed at which liquid travels along or through a textile held vertically.

In some embodiments, the one or more hydrophilic agents adhered to the textile material increase the horizontal wicking rate of the starting textile material by at least 50%, preferably 100%, more preferably 300% and most preferably 500% when tested according to AATCC test method 198-2013. The treated textile material preferably exhibits a horizontal wicking rate of at least 15 mm²/sec, preferably at least 20 mm²/sec, more preferably at least 25 mm²/sec, and most preferably at least 30 mm²/sec, when tested according to AATCC test method 197-2013. The horizontal wicking rate is the change in the area of the liquid with respect to time as the liquid travels through a textile held horizontally.

In some embodiments, the one or more hydrophilic agents adhered to the textile material have the effect that the textile material becomes noticeably more easy to squeeze out than the starting textile material, which is particularly advantageous if the textile is used together with water or other solvents.

In some embodiments, the one or more stain release agents adhered to the textile material increase the stain release rating of the starting textile material by at least one grade, more preferably by at least two grades, particularly at least three grades, and most preferably 4 grades, when tested according to AATCC test method 130-2010. The treated textile material preferably has a stain release rating, measured in accordance with AATCC test method 130-2010, of at least grade 3, preferably at least grade 4, and most preferably grade 5.

In some embodiments, the one or more stain release agents adhered to the textile material increase the stain/oil repellency rating of the starting textile material, measured in accordance with AATCC test method 118-2013, by at most one grade, preferably do not increase the stain/oil repellency. The treated textile material preferably exhibits a stain/oil repellency rating, measured in accordance with AATCC test method 118-2013, of at most grade 1, preferably grade o.

### Starting Textile Material

Generally, any textile material can be used as the starting textile material if it comprises functional groups having the ability to bond one or more antimicrobial agents to the textile material. According to some embodiments of the invention, the starting textile material comprises hydroxyl, peptide and/or carbonyl groups, in particular hydroxyl and/or peptide. These functional groups enable fixing, bonding, attaching or adhering of one or more antimicrobial and/or hydrophilic or stain release agents to the textile material. In exemplary embodiments, the starting textile material comprises peptide and/or hydroxyl groups, in particular hydroxyl groups. According to the preferred embodiments of the invention, the textile material is substantially a synthetic textile material, or at least a blend comprising a cellulosic textile material and a synthetic textile material. According to specific embodiments of the invention, the cellulosic textile material comprises cotton, viscose, rayon, linen, hemp, ramie, jute, and combinations (blends) thereof, preferably cotton or viscose or combinations thereof.

Examples of embodiments of synthetic textile material include polyester, polyamide (nylon), acrylic polyester, spandex (elastane, Lycra), aramids, modal, sulfar, polylactide (PLA), lyocell, polybutyl tetrachloride (PBT), and combinations (blends) thereof, preferably polyester. Blends of cellulosic and synthetic textile materials comprise between 20% and 80% of cellulosic textile material, preferably between 30% and 75%, more preferably between 50% and 70%, and most preferably about 60%. In specific embodiments the blend of cellulosic textile material and synthetic textile material comprises between 20 and 80% of synthetic textile material, preferably between 25 and 70%, more preferably between 30 and 50%, and most preferably about 40%.

Synthetic textiles are typically stronger and more durable than most textiles made of natural fibers. In addition, it is typically easier to bind chemical substances to a synthetic textile. Counterexamples are the antimicrobial agents PHMB and chitosan, which adhere to synthetic textiles only to a limited extent. Synthetic textiles can be designed to not wrinkle, and they typically offer a priori stretching and stain release functionality. However, they are typically hydrophobic in nature and hardly biodegradable. Depending on the requirements, these functionalities may also be disadvantageous. Natural textiles are, in turn, fairly biodegradable and hydrophilic in nature. The advantages of synthetic and natural textiles tend to be opposing. Wrinkling, stain release and hydrophobic/hydrophilic properties may be mentioned as examples.

The aforementioned textile materials may be textile material selected from the group consisting of woven, knitted, warp knitted, crocheted, and non-woven fabrics. The textile material is preferably a raised fabric, e.g. a looped fabric (such as a terry fleece or a polar fleece) or a fabric having a peach finishing or a brushed finishing. Wipes with textiles which did not undergo a raising process may leave traces, which may lead to a quick repopulation given the reproduction rate of microbes.

The skilled person understands that several of the components listed above may be combined and thereby form the starting textile material to be finished with process 10 described subsequently.

In general, any starting textile material can be processed with said process 100, wherein the textile material is a fiber, preferably a yarn or a fabric, and most preferably a fabric. In case the textile material is a fabric, it can generally have any specific weight, or fabric weight, such as e.g. 100, 200 or 300 g/m² (GSM)

### Finishing Process

### Process Cycles

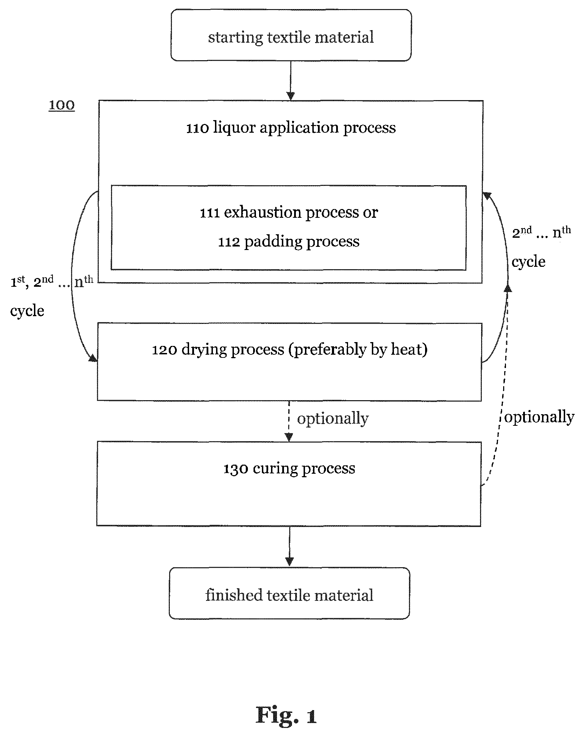

The preferred process of finishing a textile material according to the present invention comprises one or more process cycles 100 of adhering antimicrobial and/or hydrophilic/stain release agents to a textile material. Each process cycle 100, exemplarily shown in Fig. 1, can be divided into at least two process steps, a first process step 110 and a second process step 120. The first process step is a liquor application process. The liquor application process is followed by the second process step 120, in which the textile material is dried, preferably by subjecting it to a heat treatment. Optionally, the step of drying is followed by a third process step 130, in which the textile material is cured. At least the last cycle of the process of finishing a textile material according to the present invention should comprise the third step 130 of curing.

### Liquor Application Process

A liquor is a liquid containing chemicals to be applied to a textile. In the present invention, the liquor comprises one or more antimicrobial agents and/or one or more hydrophilic/stain release agents. A liquor application process is any process by which the textile is brought in contact with the liquor to treat the textile with the chemicals. The liquor application process 110 shown in Fig. 1 comprises preferably an exhaust process 111 or a padding process 112.

Preferably, the liquor has a pH-value of at most 6.9, preferably at most 6.5, more preferably at most 6.3, in particular at most 6.0, and most preferably at most about 5.5, and/or a pH-value of at least 3.0, preferably at least 3.5, more preferably at least 4.0, even more preferably at least 4.5, in particular at least 5.0, and most preferably at least about 5.5.

The liquor contains a solvent, preferably water, and the agents and the solvent preferably form a homogenous mixture, and in particular do not form a slurry or dispersion. The antimicrobial and/or the hydrophilic/stain release agents and preferably all other components in the liquor are preferably dissolved in the liquor, in particular are they not dispersed in the liquor, and/or the liquor is substantially free of solids.

The dynamic viscosity of the liquor at 20 °C and/or 80 °C, in centipoise (cP), is preferably at most 20% higher than the dynamic viscosity of water at 20 °C and/or 80 °C, respectively, preferably at most 10%, more preferably at most 5%, particularly at most 2%, and most preferably at most about 0% higher. The amount of latex in the liquor is preferably at most 10 gpl, preferably at most 5 gpl, more preferably at most 2 gpl, even more preferably at most 1 gpl, and most preferably o.

The amount of cyclodextrin and/or inclusion complexes, in particular inclusion complexes of fiber-reactive cyclodextrin derivatives and antimicrobial agents is preferably at most 10 gpl, more preferably at most 5 gpl, even more preferably at most 2 gpl, particularly at most 1 gpl, and most preferably o. The amount of dye in the liquor is preferably at most 10 gpl, more preferably at most 5 gpl, even more preferably at most 2 gpl, particularly at most 1 gpl, and most preferably o.

### Exhaustion

As is known in the art, during an exhaust process (see e.g. step 111 in Fig. 1), a textile material is brought in contact with a liquor which comprises ingredients which are transferred to the textile material during the exhaust process. This can be achieved by guiding the textile material through a container filled with the liquor. Yarn and woven fabrics are typically treated with exhaust processes. During a common exhaust process, chemicals to be applied to a textile material are dissolved or dispersed in a solvent, e.g. water, according to the required material to liquor ratio, which describes the ratio between the weight of the textile to be treated and the weight of the liquor. For example, if the desired material to liquor ratio is 1:2, there would be 600 kg of liquor for 300 kg of textile material to be exhausted. The textile material is brought in contact with the liquor, for example by immersing it into the liquor, whereby the chemicals preferably contact the fibers and more preferably enter the fibers. For obtaining proper diffusion and penetration of the chemicals in the fiber, a respective liquor temperature and respective exhaustion time are set, such that kinetic and thermodynamic reactions take place as desired. As the textile material and its fibers absorb the chemicals, the concentration thereof in the liquor decreases. As is known in the art, the degree of liquor exhaustion as a function of elapsed time is termed extent of the exhaust process. The percentage of the chemicals initially present in the liquor which is exhausted onto the textile at the end of the process is called exhaustion rate or exhaust rate. Preferably, the exhaustion rate of the exhaust process is at least 90%, preferably at least 95%, more preferably at least 98%. This exhaustion rate allows for reducing costs, as most of the ingredients of the liquor are exhausted by the textile material. It is also more ecological than processes with lower exhaustion rates.

The exhaust process 111 shown in Fig. 1 may be performed by any suitable technique, and on any suitable machine, like a yarn dying machine, a beam machine, a winch machine, a jet-dyeing machine, a continuous dyeing range (CDR), continuous bleaching range (CBR), or a jigger machine. In a jigger machine, an open-width fabric revolves on two main rollers. The fabric passes from one roller through the liquor bath at the bottom of the machine and then onto a driven take-up roller on the other side. When all the fabric has passed through the bath, the direction is reversed. Each passage is called an end. The process typically involves an even number of ends. The liquor bath has one or more guide rollers around which the cloth travels. During the immersion, the desired contact with the process liquor is achieved. When passing through the liquor bath, the fabric picks up an adequate quantity of liquor, excess of which is drained out, but still a good quantity is held in the fabric. During rotation of the rollers, the chemicals contained in the liquor penetrate and diffuse into the fabric. The largest part of the diffusion process takes place not in the liquor bath but when the fabric is on the rollers, since only a very small length of fabric is in the liquor bath at a given time, and the major part is on the rollers. Jigger machines are preferred because they are very economical and because they can be used with a high material to liquor ratio.

The exhaust process 111 shown in Fig. 1 allows for evenly spreading the liquor across the entire cross section of the textile material, such that preferably no spot of the textile material is left untouched by the liquor. As a result, interactions and/or bonds may be created between the textile material and one or more agents at this time. Preferably, most of the one or more agents of the liquor are exhausted evenly onto the entire cross section of the textile material.

In general, more heat on the fabric is better for bonding. Therefore, preferably, the temperature of the liquor during the exhaust process is sufficiently high and the exhaust time is sufficiently long such that the one or more agents in the liquor are substantially uniformly dispersed across the cross section of the textile material as a result of the exhaust process. Thus, the temperature of the liquor should be sufficiently high and the exhaust time should be sufficiently long such that preferably the textile material is well impregnated and the one or more agents are dispersed throughout the entire textile material. Preferably, the exhaust time is sufficiently long and the temperature of the liquor during the exhaust process is sufficiently high such that the textile material can achieve the desired performance after a respective curing process, as will be outlined below.

However, too much heat causes yellowness and weakens the fabric. Therefore, preferably, the temperature of the liquor during the exhaust process is sufficiently low and/or the exhaust time is sufficiently short such that the textile material does not discolor and/or turn yellow and/or its breaking (tensile) strength is not reduced by more than 15%, preferably not more than 10%, more preferably not more than 7%, and most preferably not more than 5%, as a result of the exhaust process. At too high temperatures, too much steam forms, reducing the efficiency of the process. Furthermore, if the temperature of the liquor is too high, turbulences can occur within the liquor bath and the textile material may get harmed.

The term exhaust time when used in the context of the present invention is preferably defined as the period starting when at least part of the entire batch of textile material first comes into contact with the liquor and lasting until the last part of the batch is taken out of the liquor. For a given application, the ideal exhaust time can vary significantly. In case the textile is a fabric, it will depend on the type of machine, the size of the liquor bath, and the length and weight of the fabric. For example, if the ideal exhaust time for a fabric of a length of 1,500 meters is 60 minutes, the ideal exhaust time for a fabric of a length of 3,000 meters may be 100 minutes under otherwise identical conditions. Whenever an exhaust time is specified herein, it refers to the time which is equivalent to the exhaust time of a fabric of 1,500 meters in length and 200 g/m² in weight on a standard jigger machine (e.g. model number Y₁₁₀₀ manufactured by Yamuda) being operated at a standard fabric speed (e.g. 50 meters/minute). For any given textile material and exhaustion machine, the skilled person, using common general knowledge, will be able to determine the exhaust time which is equivalent to an exhaust time specified for the above-mentioned parameters.

The breaking strength may be measured with any suitable technique, and is preferably measured in accordance with ASTM standard D 5035-11(in case the textile material is a fabric), or in accordance with ASTM standard D 2256/D 2256M-10e1 (in case the textile material is a yarn).

The inventors found that the preferred temperature of the liquor during the exhaust process and the exhaust time is substantially independent of the weight and the type of the textile material, and of the agents in the liquor. This is because the ideal exhaust process parameters are determined by the way textiles, in particular multifilament yarns and fabrics, behave in general.

In the preferred embodiments, the liquor has a temperature of at least 45 °C, preferably of at least 50 °C, more preferably of at least 60 °C, particularly of at least 70 °C, and most preferably of at least about 80 °C. Furthermore, the liquor has a temperature below boiling temperature, preferably of at most 95 °C, more preferably of at most 90 °C, particularly of at most 85 °C, and most preferably of at most about 80 °C. The exhaustion process time is preferably at most 90 min, preferably at most 80 min, more preferably at most 70 min, and most preferably at most about 60 min. Furthermore, the exhaustion process time is preferably at least 45 min, preferably at least 50 min, more preferably at least 55 min, and most preferably at least about 60 min. As shown in International patent application number PCT/EP2016/054245 by the same applicant, when a textile is treated at a temperature of 80 °C for 60 minutes, it expands and opens up, exposing individual fibers so that the agent can reach even the most remote spot, and there is even dispersion of the agents. Accordingly, different textile materials can easily be treated by means of the exhaust process 111 shown in Fig. 1 without having to change parameters of the exhaust process, while still obtaining the best possible results. Preferably, during the exhaust process 111 shown in Fig. 1, the liquor is stirred. The stirring should be performed at intervals, in other words, the stirring is performed regularly during the exhaust process with interruptions. It will be appreciated that other suitable intervals may preferably be set, depending on the specific application. Ideally, the stirring is performed continuously during the exhaust process. This intermixing of the chemicals in the exhaust bath increases reliability of the exhaust process, as one or more agents are more evenly distributed in the bath and as a result, a product with even quality throughout the entire textile material can be obtained. Preferably, the stirring is performed by means of a circulation pump, which circulates the liquor inside the exhaustion bath and which is typically comprised by a conventional exhaustion machine. The stirrer used by the inventors is a simple mixer, which is similar to but larger than a standard household mixer. The stirrer was added by the inventors to the exhaustion machine they used as it is not provided by conventional exhaustion machines. Most preferably, the liquor is stirred by means of both a circulation pump and a stirrer. Due to this extensive mixing of the liquor, the exhaust process is supported and one or more agents are well dispersed across the cross section of the textile material during the exhaust process. As is known in the art, an exhaust process is typically applied for dyeing cloth, for example. In such applications, typically only a circulation pump is applied for ensuring proper fluid characteristics of the bath, such that a homogeneous dispersion of the dyeing molecules is present in the bath. However, since the one or more agents used in the context of the present invention can be less soluble in water compared to dyeing agents, the utilization of both a stirrer and a circulation pump assures that the agents are not undissolved and do not settle at the bottom of the bath. Instead, due to the combination of both stirring means, the agents are uniformly and homogeneous dispersed throughout the bath.

Accordingly, with exhaust process 111 shown in Fig. 1, agents are substantially uniformly dispersed across the cross section of the textile material, whereby the textile material itself, advantageously, does not yellow and essentially does not lose its breaking strength.

### Padding

Any suitable technique can be utilized for performing a padding process 112 shown in Fig. 1, in which preferably a respective liquor is prepared and fed through a pump to a respective padding mangle. Accordingly, padding process 112 shown in Fig. 1 preferably comprises applications of one or more rolls to obtain optimum wet pickup of the liquor on the textile material. According to a preferred embodiment, the textile material passes through a multiple trough padding mangle, or a continuous dyeing or bleaching range. The liquor may be at room temperature or it may be heated during the padding process.

The appropriate padding mangle pressure is typically predetermined, depending on the quality of the textile material, and it is in general set such that the pick-up rate (or "wet pick-up") of the agents is optimized. The pick-up rate specifies the amount of liquor applied and is defined as a percentage on the weight of the dry untreated textile as follows: % pick-up rate = weight of liquor applied x 100 / weight of dry textile. For example, a pick-up rate of 65% means that 650 grams of liquor are applied to 1 kg of textile. Preferably, a liquor pickup rate of the padding process is at least 30%, preferably at least 40%, more preferably at least 50%, particularly at least 60% or at least 80%, and most preferably at least about 100%, and/or at most 140%, preferably of at most 130%, more preferably of at most 120%, particularly at most 110%, and most preferably of at most about 100%. However, if the textile is already to a certain extent saturated with agents before the padding process is applied, e.g. because another process cycle has been performed previously, it is believed that the effective pick-up rate for the agents is only about 70% of the nominal pick-up rate mentioned above, in the sense that the rest of the agents padded onto the fabric does not become permanently fixed to the fabric.

### Drying

Any suitable technique can be utilized for performing the drying process step 120 shown in Fig. 1. Drying is, however, preferably performed by subjecting the textile material to a heat treatment. The heat treatment is preferred because it is more efficient for various reasons: accelerated manufacturing, i.e. shortened residence time of the textile material in the production chain and lean process management without removal for drying purposes and re-insertion of the textile material in the processing chain may be mentioned as examples. Furthermore, the textile should be dried by a heat treatment before it is washed. This is because the heat treatment will achieve basic bonding of the agents to the textile so that they will not immediately be washed out during the washing process.

After drying process 120 by heat treatment, the textile material should be 99% devoid of moisture. However, when the textile cools down to room temperature, it will have a moisture regain of, e.g., about 7-8% for cotton and of about 4-5% for polyester.

Preferably the drying is conducted at least partially at an ambient temperature of at least 70 °C, preferably at least 100 °C, more preferably at least 110 °C, most preferably at least about 120 °C. Lower temperatures would require longer dwell time, which is disadvantageous because a longer dwell time has a negative impact on the textile in terms of yellowing and also on the strength of the fabric. Furthermore, the drying is preferably conducted at an ambient temperature of at most 160 °C, preferably of at most 140 °C, more preferably of at most 130 °C, and most preferably of at most about 120 °C.

Preferably, the drying time at the temperatures given above is of at least 30 seconds, preferably at least 40 seconds, more preferably at least 50 seconds, and most preferably at least about 60 seconds, per 100 g of fabric weight per m² (in case the textile material is a fabric). Further preferably, the drying is performed over a period of at most 120 seconds, preferably at most 90 seconds, more preferably at most 75 seconds, most preferably at most about 60 seconds, per 100 g of fabric weight per m² (in case the textile material is a fabric). It will be appreciated that the drying times increase with increasing fabric weight (per m²). The skilled person understands that similar drying times apply if the textile material is a yarn, and understands to choose respective drying times which then depend on the yarn diameter.

Drying process 120 is typically conducted by passing the textile material through a stenter or stenter frame (sometimes also referred to as a "tenter") or similar drying machine. By drying the textile material, preferably excess moisture is removed.

### Curing

A curing process in the context of the present invention (see e.g. 130 in Fig. 1) is essentially a heat treatment process applied to a textile material for inducing physical cross-linking. Curing can only be performed once the textile is dry because the temperature of the textile cannot exceed 100 °C until the water in the textile is evaporated.

The curing temperature is preferably sufficiently high and the curing time is preferably sufficiently long such that one or more agents applied to (preferably exhausted and/or padded onto) the textile material are sufficiently strongly fixed or bonded to the textile material. They should preferably be set such that the agents are bound to the textile material and optionally polymerized, become an inherent part of the textile material and provide the desired properties of the textile material in a wash-durable or even non-leaching manner. Depending on the agents and chemicals used, also a large part of the crosslinking of the agents takes place during the curing step. In case the textile material is a fabric, the curing time depends on the weight of the fabric (per m²). However, the inventors found that the preferred curing temperature, which will be detailed below, is substantially independent of the type of the textile material.

Furthermore, the curing temperature is sufficiently low and the curing time is sufficiently short such that the textile material does not discolor and/or turn yellow, and/or its breaking strength is not significantly reduced. Further preferred, the curing temperature is sufficiently low and the curing time is sufficiently short such that the textile material does not melt and/or burn and/or yellow, and/or that the colors of the textile material do not substantially change (discolor) as a result of the curing.

In the preferred embodiments, the curing process is conducted at least partially at an ambient curing temperature of at least 140 °C, preferably at least 160 °C, more preferably at least 170 °C, particularly at least 175 °C, and most preferably at least about 180 °C. Preferably, the curing process is conducted at an ambient temperature of at most 200 °C, more preferably at most 190 °C, even more preferably at most 185 °C, and most preferably at most about 180 °C. Thus, in the most preferred embodiment, the maximum curing temperature is 180 °C, independent from the textile material treated with process 100.

In particular where the textile material is a fabric of less than 350 grams per m², curing takes place at the above stated curing ambient temperatures over a period of preferably at least 30 seconds, preferably at least 40 seconds, more preferably at least 50 seconds, most preferably at least about 60 seconds, and preferably over a period of at most 120 seconds, preferably at most 90 seconds, more preferably at most 80 seconds, particularly at most 70 seconds, most preferably at most about 60 seconds.

Where the textile material is a fabric between 350 and 500 grams per m², curing takes place at the above stated curing ambient temperature over a period of preferably at least 45 seconds, preferably at least 60 seconds, more preferably at least 75 seconds, most preferably at least about 90 seconds, and preferably over a period of at most 180 seconds, preferably at most 160 seconds, more preferably at most 140 seconds, particularly at most 120 seconds, most preferably at most about 90 seconds.

Where the textile material is a fabric of at least 500 grams per m², curing takes place at the above stated curing ambient temperature over a period of preferably at least 60 seconds, preferably at least 75 seconds, more preferably at least 90 seconds, most preferably at least about 120 seconds, and preferably over a period of at most 240 seconds, preferably at most 210 seconds, more preferably at most 180 seconds, particularly at most 150 seconds, most preferably at most about 120 seconds.

The preferred process parameters for curing (at 180 °C during the curing times mentioned above) were obtained in an extensive research and development campaign performed by the inventors and described in International patent application PCT/EP2016/054245 by the same applicant.

The curing process 130 is preferably carried out in a pass through a stenter. Referring to Fig. 1, a drying process 120 following a liquor application process may be directly followed by a curing process 130. In this case, the drying process 120 and the curing process 130 is preferably carried out together with one single pass through the stenter, preferably with a temperature ramp-up and ramp-down procedure as described in International patent application PCT/EP2016/054245 by the same applicant. Furthermore, where the textile material is a fabric, drying and curing of the textile material are performed at the above stated drying/curing ambient temperatures over a period of together at least 45 seconds, preferably at least 50 seconds, more preferably at least 55 seconds, most preferably at least about 60 seconds, per 100 grams of fabric weight per square meter, and preferably at most 75 seconds, preferably at most 70 seconds, more preferably at most 65 seconds, most preferably at most about 60 seconds, per 100 grams of fabric weight per square meter.

### Putting the cycles together

The subsequent sections are dedicated to the description of the preferred and most preferred embodiments based on the above stated elementary building blocks specifying the starting textile materials, composition of liquors as well as processing parameters including concentrations of agents, temperatures and processing times at standard pressure.

Exhaustion is preferred over padding in the first process cycle if the starting textile material is a multifilament yarn or a fabric made out of them, which is preferred for most applications because multifilament textiles are stronger, have a higher surface area, and can be blended. In an exhaust process, the multifilament textile opens up and the fibers are individually exposed to penetration by the agents. Thus, by use of an exhaust process, the agents can diffuse into the fibers and do not occupy the surface space of the fibers to the same extent as it is the case in more superficial liquor application processes like padding or spraying.

The use of an exhaust process in the first process cycle is particularly advantageous in cases where the first process cycle is followed by a further process cycle. The use of an exhaustion process in the first process cycle allows to improve the performance or convey another property by a second process cycle, in particular by a second process cycle in which a padding process is used. E.g., the inventor found in experiments, whose results are not reproduced herein, that the antimicrobial performance and/or the wash-durability thereof can be increased if the textile material is treated with an antimicrobial liquor application process in each of two process cycles. In this case, the first antimicrobial liquor application process is preferably an exhaust process, and the second liquor application process is preferably a padding process. The two antimicrobial cycles can then be followed by one or more cycles in which a hydrophilic/stain release liquor application process is performed.

In contrast, repeated superficial liquor applications like repeated padding applications will not improve performance, or at least not to the same extent, and it will be more difficult to impart another property in the second cycle. Furthermore, the inventors found that leaching is at lowest values only when exhaustion is used in the first process cycle. On the other hand, in the case of non-woven fabrics, padding is preferred because non-woven fabrics can oftentimes not withstand the forces applied by exhaustion machines like jiggers.

For embodiments which make use of more than one processing cycle, padding is preferred for all subsequent cycles, independent of the starting textile material. This is because once a textile has been exhausted with agents in a first process cycle, the interior of the fibers is saturated at least to a certain degree, and space for more agents to be adhered to the textile material will be found primarily on the fiber surface. Furthermore, padding is less time consuming and therefore less costly than exhaustion.

Curing, which consumes a lot of energy and decreases the breaking strength of the textile considerably, is preferably only performed in the last cycle. This is in general sufficient for bonding even the agents applied in the previous cycles to the textile. Some embodiments may comprise a washing step at the end of one or more or even all cycles, to obtain particularly low leaching values (for a detailed description see International patent application PCT/EP2016/054245 by the same applicant). However, this may not be necessary for most of the preferred applications disclosed herein, such as wipes and other sanitary products. Where washing is carried out after the drying step, in particular without or before curing, the drying should be performed with a heat treatment, preferably at least at the above-specified temperatures for drying. The heat treatment will ensure that there is a basic bonding between the textile and the agents applied in this cycle, which prevents washing out of these agents in the washing step.

As a rule, where both antimicrobial and hydrophilic/stain release agents are applied to the starting textile material, antimicrobial and hydrophilic/stain release agents are, on the one hand, separately applied, i.e. in separate cycles. On the other hand, the antimicrobial agents are applied in one or more cycles prior to the cycle in which hydrophilic/stain release agents are applied, or applied for the first time. The inventor found that it is difficult, if not impossible to apply antimicrobial agents and hydrophilic/stain release agents in the same liquor in a wash-durable manner, or to apply antimicrobial agents in a wash-durable manner after hydrophilic/stain release agents have been applied to the textile. Metal, in particular silver is an exception to this rule, as it may be applied together with hydrophilic/stain release agents in the same liquor, i.e. in the same cycle. However, it should not be applied after the application of hydrophilic/stain release agents, as the pores of the textile would be blocked and bonding would be severely hampered.

A first group of embodiments of the present invention comprises the finishing of starting textile materials by adhering only antimicrobial agents to them but no hydrophilic/stain release agents, in one or more cycles of the finishing process of the present invention. A second group of embodiments comprises the finishing of starting textile materials by adhering only hydrophilic/stain release agents to them but no antimicrobial agents, in one or more cycles of the finishing process of the present invention. However, the most preferred group of embodiments of the present invention comprises the application of both antimicrobial agents and hydrophilic/stain release agents, in two separate cycles.

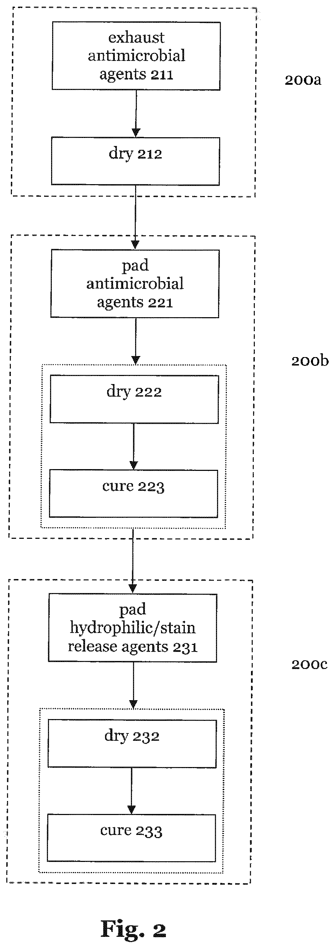

The preferred process of adhering both antimicrobial agents and hydrophilic/stain release agents to a textile material will now be described with reference to Fig. 2. In a first cycle 200a, the starting textile material, preferably a woven fabric, is subjected to an exhaustion process 211, wherein the liquor comprises one or more antimicrobial agents dissolved in water, with the pH-value adjusted as specified above. The selection and the concentration of the antimicrobial agents in the liquor are such that the exhaustion process 211 results in a textile material to which the agents as specified above are adhered in the combinations and the amounts as specified above. Exhaustion 211 is performed at standard pressure with an exhaustion temperature of 80 °C for 60 minutes while the liquor is stirred. The first process cycle 200a is finished by drying 212 as described above, at 120 °C.

In a second cycle 200b, the textile material is subjected to a padding process 221, wherein the liquor comprises one or more antimicrobial agent(s) dissolved in water, with the pH-value again adjusted as specified above. The selection and the concentration of the hydrophilic/stain agents in the liquor are such that the padding process 221 results in a textile material to which the agents are adhered in the combinations and the amounts as specified above. Padding 221 is performed at both standard pressure and temperature, with a liquor pickup rate of 100%. The second process cycle is finished by drying 222 followed by curing 223 at 180 °C, wherein drying and curing are performed in one single pass through a stenter.

In a third cycle 200c, the textile material is subjected to another padding process 231, wherein the liquor comprises hydrophilic/stain release agents dissolved in water, with the pH-value again adjusted as specified above. The selection and the concentration of the hydrophilic/stain agents in the liquor are such that the padding process 231 results in a textile material to which the agents are adhered in the combinations and the amounts as specified above. Padding 231 is performed at both standard pressure and temperature, with a liquor pickup rate of 100%. The third process cycle is finished by drying 232 followed by curing 233 at 180 °C, wherein drying and curing are performed in one single pass through a stenter, which provides the finished textile material.

It will be appreciated that one or more additional process steps or cycles may be introduced between the individual process steps or cycles of process 100 of Fig. 1. Furthermore, one or more additional process steps or cycles may be performed prior to or after performing process 100 of Fig. 1. For example, before the start of process 100 with the liquor application process 110, the textile material should be tested, washed and/or cleaned. Preferably, the fabric is first tested and if necessary washed or cleaned, so that the fabric is free from all chemical contaminants that would hinder the application of the chemistry to the textile. In a particularly preferred embodiment, one or more of the following steps may be performed prior to conducting process 100 of Fig. 1: Testing the textile material at laboratory scale to verify and confirm that it meets respective selection criteria, batching and stitching together of individual textile pieces on a frame, inspecting the textile material thoroughly for defects, ensuring that the fabric is free from any chemical contaminants.

The textile material may be dyed prior to performing process 100. In some embodiments, the textile material is manufactured to be multifunctional. After having performed process 100, i.e. after the antimicrobial and/or hydrophilic/stain release treatment, a respective multifunctional treatment is performed. With such a multifunctional treatment, the textile material may be provided e.g. with UV-blocking and/or other properties. It is also possible to conduct a multifunctional treatment in a padding process 112, wherein the padding liquor contains the respective functional agents, in addition to antimicrobial or hydrophilic/stain release agents.

It will be appreciated that different machines may be utilized in case the textile material is a yarn. For example, the exhaustion process may be performed with a pressurized yarn dyeing machine, and the yarn may then be treated with a hydro extractor for removing excess moisture. The drying and curing of the yarn may take place in a Radio Frequency RF Dryer and curing machine. The dwell times thereby depend on the yarn diameter, wherein the temperatures mentioned above still apply.

### EXAMPLES

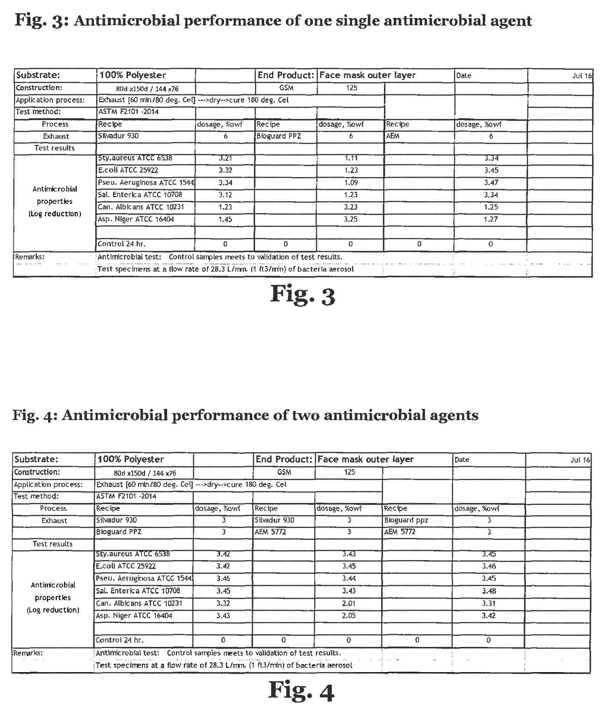

A series of different textile materials was finished and the finished textile materials, different multi-layer filter materials and different face masks were tested according to the test protocols stated herein. The finishing process and the test results as well as the references to the underlying test protocols are summarized in the tables of Fig. 3 to Fig. 12. All finished textile materials were treated with antimicrobial agents and can be used for multi-layer filter materials and face masks. The multi-layer filter material was assembled sandwiching one to four layers of particulate filter material between two layers of finished textile material. The layers were attached to each other in a rectangular shape of about 144 mm x 76 mm using ultrasonic welding forming a pad. The pad was equipped with straps on all four corner of the pad thus forming a face mask that can be installed over mouth and nose of a wearer.

The finishing processes are summarized on the upper side of each table under the section "application process". Fabrics were produced under laboratory conditions closely simulating the finishing processes described below.

### Chemicals

Chemicals used for the examples and mentioned in the tables comprise the following:
"Bioguard PPZ" (=Bioguard PPZ 250) of Beyond Surface Technologies, Switzerland: a solution containing 25% of propiconazole;
"Chitosan-102" of Go Yen Chemical, Taiwan: a solution containing 15% of chistosan;
"Silvadur 930" of Dow Chemical Company, USA: a solution containing 0.17% of silver cations trapped in a matrix;
"AEM 5772" of Aegis, USA: a solution containing 72% of quaternary ammonium organosilane compounds;
"Hydrosil" (= Hydrosil 8900) of Britacel Silicones Ltd., India: a solution containing 30% organosilane terpolymer;

Recipes are stated in the figures. Percentages refer to the weight of the chemicals relative to the weight of the textile material ("o.w.f.") if not otherwise stated. The percentages of active agents which were used can be calculated with the concentration of actives in the chemicals as specified above. E.g., 6% Bioguard PPZ means that 25% x 6% = 1.5% o.w.f. of Bioguard PPZ were used. The expression "gpl" refers to the amount of a chemical in a liquor. Again, the amount of actives can be calculated with the concentration of actives in the chemicals as specified above. E.g., 50 gpl Hydrosil means that 30% x 50 gpl = 15 gpl of organosilane terpolymer were comprised in a liquor. Other units are explicitly stated in each table.

### Test protocols

The underlying test protocols AATCC 100-2012, ASTM 2149-2010, ASTM F2101-2014, and IS9473:2002 are generally publically available from the American Association of Textile Chemists and Colorists (AATCC), the American Society for Testing and Materials (ASTM) and the Bureau of Indian Standards (IS), respectively.

The ASTM F2101-2014 was conducted as follows. The test organism used was Staphylococcus aureus (ATCC 6538), and the inoculum size was 6.5 x 10⁶ CFU/ml. An aerosol challenge apparatus with nebulizer assembly and cascade impactor was used as test equipment. The flow rate of the cascade impactor was set to 1 Cu. Ft. /min. The bacterial aerosol size was 3 ± 0.5 µm. The face mask was clamped between the cascade impactor and an aerosol chamber at a distance of 15 cm. The bacterial aerosol was generated in the chamber using the nebulizer assembly and drawn through the test material using a vacuum attached to the cascade impactor. Positive control samples refer to the collected aerosol with no test specimen to determine the upstream aerosol counts. As a negative control for the test specimen an impermeable polyethylene sheet was used. The ratio of upstream to downstream aerosol counts was reported as Bacterial Filtration Efficiency (BFE, in percentage). Stated results present the mean of three independent readings.

All microbiological tests were performed with cultures of the bacteria *Escherichia coli* American Type Culture Collection (ATCC) 25922, *Staphylococcus aureus ATCC* 6538, *Pseudomonas aeruginosa* ATCC 15442, *Salmonella enterica* ATCC 10708, and of the fungi *Candida albicans* ATCC 10231, and *Aspergillus Niger* ATCC 10231.

### Working Example

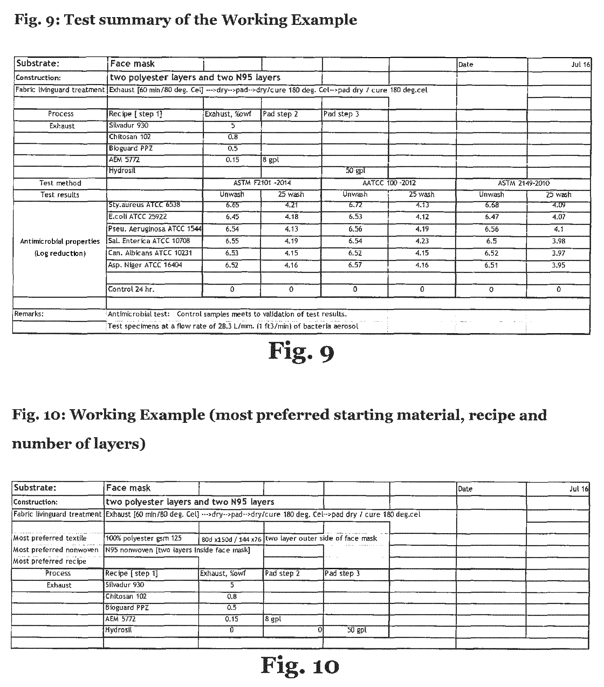

Fig. 9 to 12 summarize the preferred starting textile material, its finishing processes and recipes used for preparation, the particulate filter material, and the construction of the multi-layer filter material as used in the resulting face masks, as well a comparison of the bacterial filtration efficiency obtained by the Working Example and by a competitor face mask.

The starting textile of the Working Example was a 100% polyester woven fabric, 80 D x 150 D, 144 mm x 76 mm, fabric weight 125 g/m².

In a first process cycle, the fabric was exhausted and then dried. In a second process cycle, the fabric was padded, then dried again and cured. In a third process cycle, the fabric was again padded, then again dried and finally cured. For a more detailed description of the most preferred embodiment of the finishing process, we refer to the sections above.

In the exhaustion process, 1,500 meters (303.75 kg) of starting textile material were loaded on a jigger machine (Yamuna, model number Y1100). Into about 600 liters of water (material to liquor ratio of about 1:2), 15.2 kg (5% of the weight of the textile material) of Silvadur 930, resulting in an amount of 0.0085% silver o.w.f., 1.5 kg (0.5% of the weight of the textile material) of Biogard PPZ, resulting in an amount of 0.13% propiconazole o.w.f., 0.45 kg (0.15% of the weight of the textile material) of AEM5772, resulting in an amount of 0.1% o.w.f. quaternary ammonium organosilane, 2.4 kg (0.8% of the weight of the textile material) of Chitosan-102, resulting in an amount of 0.12% o.w.f. chitosan. The pH of the liquor was adjusted with 0.03 gpl of citric acid and maintained between pH 5 and pH 6, preferably at pH 5.5. The temperature of the liquor was set to 80 °C.

The jigger machine was started and run at a speed of 50 m/s, and the run was continued for the next 60 minutes (2 ends, with a break of less than 30 seconds between the ends). The liquor was constantly stirred with a stirrer at a speed of 300 rpm throughout the exhaustion process. The exhaustion rate was about 98%. Following this, the process bath was drained and the textile material was immediately transported to a stenter machine for drying. Thus, the exhaust time was 60 minutes.

The textile was dried by passing it through the stenter, which had 8 chambers and a length of 24 meters, at a speed of 20 centimeters per second. The maximum temperature of 180 °C was applied in all 8 chambers, i.e. during 120 seconds.

Following this first process cycle, a second process cycle with a padding process was performed to apply AEM 5772: To about 400 liters of water, 3.2 kg (8 gpl) of AEM 5772 were added, resulting in a concentration of 5.7 gpl quaternary ammonium organosilane in the liquor. During the padding process, the liquor had a temperature of 30 °C. The padding mangle pressure was 2 bar. The nominal pick-up rate was 100%. Estimating that as mentioned above, after the first process cycle, the effective pick-up rate in a padding process of subsequent process cycles is about 70% of the nominal pick-up rate, the amount of quaternary ammonium organosilane adhered to the textile material was about 0.4% o.w.f.

The textile material was then dried and cured for in total 2 minutes in a single pass through the stenter, at a maximum temperature of 180 °C. The maximum curing temperature was applied for 60 seconds (in 4 of the 8 chambers of the stenter).

Following this second process cycle, a third process cycle with a padding process was performed to apply a hydrophilic/stain release agent: To about 400 liters of water, 20 kg (50 gpl) of Hydrosil were added, resulting in a concentration of 15 gpl organosilane terpolymer in the liquor. During the padding process, the liquor had a temperature of 30 °C. The padding mangle pressure was 2 bar. The nominal pick-up rate was 100%. Estimating that as mentioned above, after the first process cycle, the effective pick-up rate in a padding process of subsequent process cycles is about 70% of the nominal pick-up rate, the amount of organosilane terpolymer adhered to the textile material was about 1.05% o.w.f.

The textile material was then again dried and cured for in total 2 minutes in a single pass through the stenter, at a maximum temperature of 180 °C. The maximum curing temperature was applied for 60 seconds (in 4 of the 8 chambers of the stenter).

The multi-layer filter material was prepared from two outer layers of the finished textile material and two N95-rated particulate filter material as inner layers. The layers were attached or seamed to each other in rectangular size of a face mask, namely 144 mm x 77 mm, using ultrasonic welding. Subsequently, the multi-layer filter material was cut just outside the boundaries of the seam.

Straps were attached at each edge of the multi-layer filter material creating a face mask that can be installed over a wearer's mouth and nose.

Different tests regarding the antimicrobial efficiency of the face mask of the Working Example were performed, the results of which are summarized in the tables of Fig. 9,10 and 11.

The face mask showed to be highly antimicrobial, and the imparted antimicrobial properties proved to be sufficiently wash-durable.

The results of the ASTM 2149-2010, the ASTM F2101-2014 and the AATCC 100-2012 test methods of the unwashed face mask showed that the face mask is capable of reducing all of the above-mentioned organisms by more than 99.9999% (6 log). Wash-durability of the antimicrobial properties of the face mask was shown after 25 standard washes as described above. Here, the antimicrobial capability of the extensively washed face mask slightly decreased to > 4 log reduction (Fig. 9).

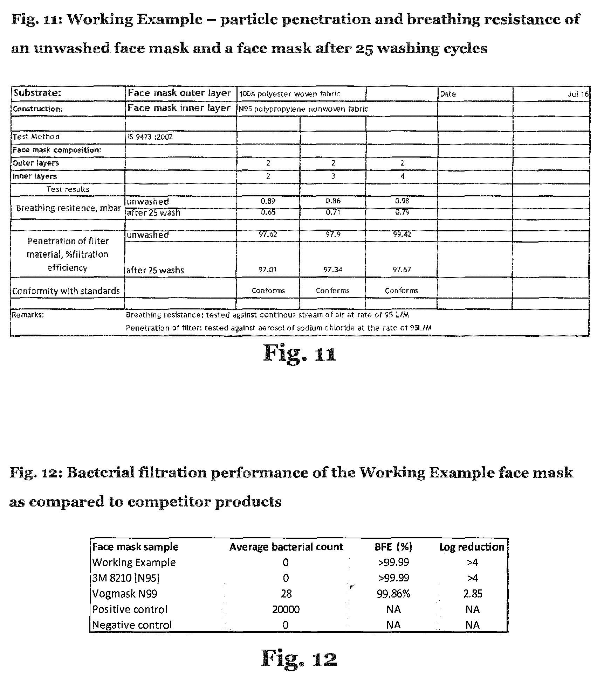

The inventors identified the "Vogmask N₉₉" as the closest competitor product on the market, which is a re-usable and washable face mask, however without antimicrobial properties. The "3M 8210 [N₉₅]" mask is another related product, however this mask is disposable, i.e. not washable. Both, the Vogmask N₉₉ and the 3M 8210 [N₉₅] mask, belong to the best-performing face masks in terms of filter efficiencies that are available on the market. In a comparative study (Fig. 12), the face mask of the Working Example, the Vogmask N99, and the 3M 8210 [N95] face mask were subjected to the ASTM F2102-2014 test, which is explained in detail above. The face mask of the Working Example performed significantly better (> 4 log reduction) in filtering the bacterial aerosol compared to the tested Vogmask N99 (2.85 log reduction). The 3M 8210 [N95] face mask performed equally well as the Working Example. This demonstrates that the washable, re-usable, and antimicrobial face mask disclosed in this invention fulfills high standards in terms of bacterial filtration efficiency and safety, with the environmental and cost benefits of a re-usable product.

### Experimental Examples

The inventors of the present invention performed comprehensive further experiments to determine the effect of different recipes, finishing processes and face mask constructions.

### Experimental Examples 1: Different combinations of antimicrobial agents

With Experimental Examples 1, the effect of the application of different numbers of agents to the starting textile materials was studied. The agents applied to the polyester material were taken from the group of silver, quaternary ammonium organosilane, and propiconazole, which are the three most preferred antimicrobial agents for treating polyester textile materials.

The textiles were treated with (1) each of the agents from the respective group individually, (2) all three tuple combinations of agents from the respective groups, and (3) all three agents from the respective groups together. The total amount of the chemicals in all experiments was 6% o.w.f. Thus, where one single agent was applied to the textile, 6% o.w.f. of the respective chemical was used, where a combination of two agents was applied, 3% o.w.f. of each of the respective two chemicals was used, and where a combination of three agents was applied, 2% o.w.f. of each of the respective three chemicals was used.

The finishing process was as described above in the context of the Working Example, but since no hydrophilic/stain release agent was applied, there was only one process cycle. The fabric was exhausted and then dried and cured in one single pass through the stenter, with the process parameters as described above.

Fig. 3 shows the antimicrobial performance of the polyester textiles treated with one single agent. The results indicate that quaternary ammonium organosilaneand silver applied individually are about equally efficient for killing bacteria (*Escherichiα coli, Staphylococcus aureus, Pseudomonas aeruginosa,* and *Salmonella enterica*)*.* However, they perform poorly when it comes to killing fungi (*Candida albicans* and *Aspergillus Niger).* On the other hand, propiconazole is efficient for killing fungi, but not efficient for killing bacteria.

Fig. 4 shows the antimicrobial performance of the polyester textiles treated with combinations of two agents. The textile which was not treated with propiconazole (i.e. the textile treated with silver and quaternary ammonium organosilane) performed poorly against fungi. On the other hand, the textiles treated with propiconazole and either silver or quaternary ammonium organosilane performed well against both bacteria and fungi. The overall performance of the textiles treated with combinations of two agents was slightly higher than the performance of the textiles treated with one single agent.

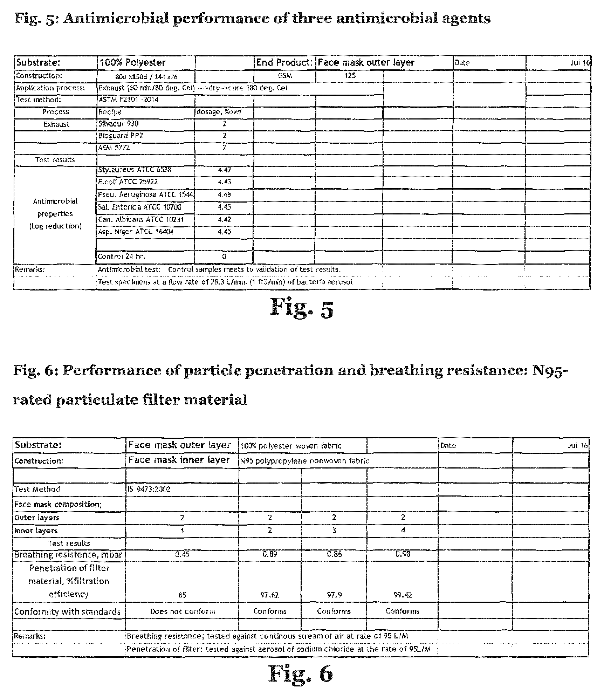

Fig. 5 shows the antimicrobial performance of the polyester textiles treated with three agents (propiconazole, silver, quaternary ammonium organosilane). The overall performance of the textiles treated with three agents was slightly higher for all microbial organisms than the performance of the textiles treated with two agents.

From Experimental Examples 1, it can be concluded that all antimicrobial agents identified as preferred by the inventors are about equally powerful against bacteria. This was also found in earlier experiments conducted by the inventors and discussed in International patent application PCT/EP2016/054245 by the same applicant. textile. Only propiconazole (or other azole-based agents) or combinations comprising propiconazole show a good performance against fungi. The experiments show a synergistic killing effect for the same organism with a growing number of agents.

### Experimental Examples 2: Particle penetration and breathing resistance

The purpose of Experimental Examples 2 was to study the effect of differently rated particulate filter materials used in the multi-layer filter material of the face masks with regard to particle penetration and breathing resistance. The tests were performed according to standard test IS 9473:2002.

Fig. 6 shows the data of Experimental Examples 2, where the inner layer material of the multi-layer filter material is of N95-rated particulate filter material. Either one, two, three or four layers of such N95-rated particulate filter material was/were sandwiched by the textile material. The breathing resistance increased significantly by using more than one N95-rated particulate filter material between the two outer textile materials. The required filtration efficiency of at least 95 % was achieved by using at least two N95-rated particulate filter material between the two outer textile materials.

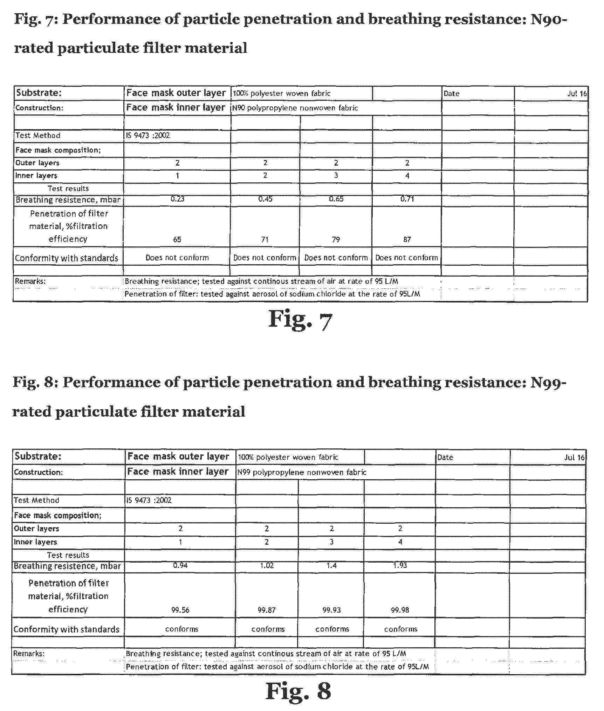

Fig. 7 shows the data of Experimental Examples 2, where the inner layer material of the multi-layer filter material is of N90-rated particulate filter material. Either one, two, three or four layers of such N90-rated particulate filter material was/were sandwiched between the textile material. The breathing resistance increased significantly by using more than one N90-rated particulate filter material between the two outer textile materials, but remained lower compared to the N95-rated particulate filter material between the two outer textile materials. The required filtration efficiency of at least 90 % was not achieved in any condition.

Fig. 8 shows the data of Experimental Examples 2, where the inner layer material of the multi-layer filter material is of N99-rated particulate filter material. Either one, two, three or four layers of such N95-rated particulate filter material was/were sandwiched by the textile material. The breathing resistance was significantly higher compared to N95-rated particulate filter material between the two outer textile materials. The required filtration efficiency of at least 99 % was achieved in any condition.

From Experimental Examples 2 it can be concluded that 2 layers of N95-rated particulate filter material are most preferably used in the multi-layer filter material, because the breathing resistance is still acceptable (0.89 mbar) and the filtration efficiency (97.62 %) well above the requirement for N95-rated particulate filter material.

### EXAMPLES FOR THE USE OF THE FINISHED TEXTILE MATERIAL AND THE MULTI-LAYER FILTER MATERIAL

### Air filters in general

Embodiments of the finished textile material and the multi-layer filter material according to the invention can be used for air-filtering purposes, such as for use in a face mask, for filters in air conditioners and other air-filtering and/or circulating systems, as well as as a filter in fresh air-inlets or exhaust air-outlets, to filter air that is stored in gas bottles, such as for scuba diving, and the like.

The multi-layer filter material of an air filter comprises two outer layers of the finished textile material, and at least one inner layer, preferably two inners layers of particulate filter material sandwiched between the two finished textile material layers.

The textile material sandwiching the particulate filter material layers, may convey stain release capability and/or antimicrobial efficiency.

In exemplary embodiments, the textile material is a woven polyester fabric. The textile material may be finished with at least one antimicrobial agent and/or at least one stain release agent.

In exemplary embodiments, the particulate filter material may be rated according to its filter efficiency as 90-, 95- or 99-rated fabric. Such particulate filter material typically is a non-woven fabric. A preferred particulate filter material is a non-woven N95-rated fabric.

The different layers of the multi-layer filter material of an air filter may be attached to each other by ultrasonic welding, stitching, gluing or by gluing using fusible interlinings. When realized by stitching, the seam of the multi-layer filter may not present the best protection against leaks. Indeed, the needle creates holes through which particulates can pass. When the seam of the multi-layer filter material of an air filter is made with glue, the seam may loosen after some wash cycles, as the glue can come out of the seam. Moreover, as it comes out, the glue can make the edges of the air filter abrasive. Hence, in the preferred embodiments, the seam is made by ultrasonic welding.

Due to the sandwiching build-up of the multi-layer filter material, with woven textile material as outer layers sandwiching the non-woven particulate filter material, the multi-layer filter material according to the present invention can be washed. This is because the rather fragile non-woven particulate filter material would easily break down, if it was not protected from mechanical forces by the woven textile material surround it.

Moreover, the multi-layer filter material of an air filter provides the advantage that due to the stain release properties it can be washed easily at mild conditions, e.g. with water only, optionally using some mild detergent, by hand or in a washing machine, while still releasing oily stain that was adsorbed or absorbed to the multi-layer filter material during earlier air-filtering activity.

The invention will be further described by the following example, which illustrates preferred embodiments without limiting the scope of the invention.

### Preferred embodiment: Face mask

A preferred embodiment of the invention is a face mask that comprises a multi-layer filter material.

### Textile material

The starting textile material may comprise at least 95%, preferably at least 97%, more preferably at least 99% of a synthetic fiber, such as polyamide (nylon), or preferably polyester. Synthetic textile materials are preferred, *inter alia,* because they can be welded with ultrasound. Further, woven textile materials are preferred in order to protect the inner, non-woven layers, and to render the face mask wash-durable.

The textile material of the multi-layer filter material has a weight of at least 50 GSM (grams per square meter), preferably at least 80 GSM, more preferably at least 100 GSM, particularly at least 120 GSM, and most preferably at least about 125 GSM. The textile material can have a weight of at most 250 GSM, preferably at most 200 GSM, more preferably at least 170 GSM, particularly at least 150 and most preferably at most about 125 GSM.

Advantageously, the textile material is equipped with antimicrobial agents. For example, an azole-based compound is adhered to the textile material in an amount of at most 1%, preferably at most 0.8%, more preferably at most 0.5%, and most preferably at most about 0.3% on weight fabric of the textile material, further in an amount of at least 0.005%, preferably at least 0.01%, more preferably at least 0.02%, particularly at least 0.05%, and most preferably at least 0.1% on weight fabric of the textile material; and/or metal is adhered to the textile material in an amount of at most 0.14%, preferably at most 0.12%, more preferably at most 0.10%, particularly at most 0.08% and most preferably at most 0.06% on weight fabric of the textile material, further in an amount of at least 0.0005%, preferably at least 0.001%, more preferably at least 0.002%, and most preferably at least about 0.005% on weight fabric of the textile material; and/or a quaternary ammonium organosilane compound is adhered to the textile material in an amount of at most 1.5%, preferably at most 1.25%, more preferably at most 1%, in particular at most 0.8 %, and most preferably at most about 0.6% on weight fabric of the textile material, further in an amount of at least 0.01%, preferably at least 0.025%, more preferably at least 0.05%, and most preferably at least about 0.1% on weight fabric of the textile material; and/or polyglucosamine is adhered to the textile material in an amount of at most 1.0%, particularly at most 0.6%, most preferably at most about 0.3% on weight fabric of the textile material, further in an amount of at least 0.01%, preferably at least 0.025%, more preferably at least 0.05%, particularly at least 0.075%, and most preferably at least about 0.1% on weight fabric of the textile material.

Advantageously, hydrophilic and/or stain release agents can be adhered to the textile material, with the types and amounts of agents selected as described in the sections above, to accelerate the distribution of fluid in the layer, and to increase its washability. The antimicrobial agents and/or the hydrophilic agents can be applied to the textile material by the process as described above.

### Particulate filter material

In exemplary embodiments, the particulate filter material may be rated according to its filter efficiency as 90-, 95- or 99-rated fabric. A preferred particulate filter material is a non-woven N95-rated fabric. Preferably two layers of such N95-rated filter material are sandwiched between two layers of finished textile material.

### Seam

The face mask can have a seam which is located at one or more edges of the face mask, where the layers of the multi-layer filter material are directly or indirectly attached together. The layers may be attached together by stitching, gluing, or preferably by ultrasonic welding. The seam can have a width of at least 2 millimeters, preferably at least 4 millimeters, more preferably at least 6 millimeters, even more preferably at least 8 millimeters, most preferably at least 1 centimeter, and/or of at most 2 centimeters, preferably at most 1.5 centimeters, more preferably at most 1.3 centimeters, most preferably at most 1 centimeter.

In a preferred embodiment of the face mask, the seam is located on one or preferably both longitudinal edges of the face mask, more preferably on all edges of the face mask. The layers are attached to each other by the seam.

### Shape of the face mask and fixing means

The face mask may have the shape of a pad, with dimensions of 5 to 15 cm (width) and 10 to 25 cm (length). At least two expansion pleats may be integrated in order to balloon over nose and face and to grant both wearing comfort and protection against breathing unfiltered air. A nose clip may be further integrated to better fit the face mask to the nose thus reducing the amount of inhaled or exhaled unfiltered air by-passing the face mask.

Fixing means to attach the face mask to the wearer's head may be provided by ear loops or straps to be fixated around the head and/or neck. Those fixing means may be adhered at each edge of the multi-layer filter material of the face mask.

Preferably, nose clip and/or the fixing means are also adhered to the multi-layer filter material by means of ultrasonic welding. Other techniques, such as stitching could result in holes that diminish the high filtration efficiency of the face mask.

## Claims

1. A face mask comprising a multi-layer filter material, wherein the multi-layer filter material comprises at least one layer of a textile material and at least one layer of particulate filter material, wherein
one or more stain release agents and/or one or more hydrophilic agents and one or more antimicrobial agents are adhered to the textile material.

2. The face mask of claim 1, wherein the one or more antimicrobial agents comprise
- at least one, preferably at least two, more preferably at least three, even more preferably at least four, or all five selected from the group consisting of a quaternary ammonium organosilane compound, metal, an azole-based compound, polyglucosamine, and polyhexamethylene biguanide; or
- at least one, preferably at least two, more preferably at least three, and most preferably all four selected from the group consisting of metal, an azole-based compound, polyglucosamine, and polyhexamethylene biguanide; or
- at least one, preferably at least two, more preferably all three selected from the group consisting of metal, an azole-based compound, and a quaternary ammonium organosilane compound; or
- at least metal and at least one selected from the group consisting of an azole-based compound, a quaternary ammonium organosilane compound, polyglucosamine, and polyhexamethylene biguanide; or at least an azole-based compound and at least one selected from the group consisting of a quaternary ammonium organosilane compound, polyglucosamine, and polyhexamethylene biguanide; or at least a quaternary ammonium organosilane compound and at least one selected from the group consisting of polyglucosamine and polyhexamethylene biguanide; or at least polyglucosamine and polyhexamethylene biguanide.

3. The face mask of claim 1 or 2, wherein the one or more stain release agents adhered to the textile material comprise at least one selected from the group consisting of fatty alcohol ethoxylates, and organosilane terpolymers, preferably organosilane terpolymers.

4. The face mask of any of the preceding claims, wherein the one or more hydrophilic agents adhered to the textile material comprise at least one selected from the group consisting of carboxylic acid esters, polyester ether copolymers, starch based emulsions, preferably fatty alcohol ethoxylates, and organosilane terpolymers, preferably fatty alcohol ethoxylates or organosilane terpolymers, most preferably organosilane terpolymers.

5. The face mask of any of the preceding claims, wherein the textile material is selected from the group consisting of woven, knitted, warp-knitted, and crocheted fabrics, preferably from a woven fabric.

6. The face mask of any of the preceding claims, wherein the textile material is a synthetic textile material comprising one or more selected from the group consisting of polyester, polyamide (nylon), acrylic polyester, spandex (elastane, Lycra), aramids, modal, sulfar, polylactide (PLA), lyocell, polybutyl tetrachloride (PBT), and combinations (blends) thereof, preferably polyester or polyamide (nylon), most preferably polyester.

7. The face mask of any of the preceding claims, wherein the particulate filter material is an at least N90/R90/P90-rated, preferably an at least N95/R95/P95-rated, and most preferably an at least N99/R99/P99-rated non-woven fabric.

8. The face mask of any of the preceding claims, wherein the multi-layer filter material comprises at least two layers, more preferably at least three layers of particulate filter material as defined in claim 8, preferably the particulate filter material being at least N95/R95/P95-rated.

9. The face mask of any of the preceding claims, wherein two layers of the textile material are used as outer layers to sandwich the at least one layer of particulate filter.

10. The face mask of any of the preceding claims, wherein the layers of the multi-layer filter material are ultrasonically welded to each other.

11. The face mask of any of the preceding claims, wherein the face mask exhibits a bacterial filtration efficiency (BFE) of at least 99.9% (3 log), preferably of at least 99.99% (4 log), most preferably of at least 99.999% (5 log) as measured in accordance with test method ASTM F2101-2014 (test organism: *Staphylococcus aureus* (ATCC 6538); inoculum size: 6.5 x 106 CFU/ml; flow rate: 1 Cu. Ft./min; aerosol particle size: 3 ± 0.5 µm; distance: 15 cm).

12. The face mask of any of the preceding claims, wherein the face mask exhibits a filter efficiency of at least 96%, preferably of at least 97%, most preferably 98% in accordance with test method IS 9473-2002, measured for the passing percentage of a sodium chloride aerosol (method option A-4.1).

13. The face mask of any of the preceding claims, wherein the one or more stain release agents adhered to the textile material increases the stain/oil repellency of the textile material, measured in accordance with AATCC test method 118-2013, by at most one rating, preferably does not increase the stain/oil repellency, more preferably decreases the stain/oil repellency, and most preferably decreases the stain/oil repellency by at least one rating.

14. The face mask of any of the preceding claims, wherein the claimed properties of the face mask, such as antimicrobial efficiency, filter efficiency and/or stain release capability, are achieved even after at least 25 laundry washes in a laundry washing machine at 85±15 °C for 10-15 minutes, preferably using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

15. The face mask of any of the preceding claims, wherein the finished textile material of the multi-layer filter material is obtained by a process comprising the steps of:
- treating the textile material using an antimicrobial liquor application process like an exhaustion or padding process, wherein the liquor comprises one or more antimicrobial agents;
- drying the textile material and treating the textile material using a stain release liquor application process like an exhaustion or preferably a padding process, wherein the liquor comprises one or more stain release agents and/or one or more hydrophilic agents; and
- subjecting the treated textile material to a heat treatment;
and wherein all layers of the multi-layer filter material, the two finished textile material layers and the at least one particulate filter material layer, are adhered to each other by means of ultrasonic welding.
